# EUROPEAN PATENT SPECIFICATION

(11) **EP 2 943 454 B1**
(45) Date of publication and mention of the grant of the patent: **18.08.2021**
(21) Application number: 14700306.5
(22) Date of filing: 10.01.2014
(51) Int. Cl.: C07C 215/54, C07C 57/145, A61K 31/137, A61P 25/04

(54) **TAPENTADOL MALEATE AND CRYSTALLINE FORMS THEREOF**
TAPENTADOLMALEAT UND KRISTALLINE FORMEN DAVON
MALÉATE DE TAPENTADOL ET FORMES CRISTALLINES ASSOCIÉES

(30) Priority: 10.01.2013 SI 201300008; 12.02.2013 SI 201300032
(43) Date of publication of application: 18.11.2015
(73) Proprietor: KRKA, tovarna zdravil, d.d., 8501 Novo mesto (SI)
(72) Inventor: KLJAJIC, Alen, 3000 Celje (SI); SIMONCIC, Ana Bergant, 8210 Trebnje (SI); PECAVAR, Anica, 8000 Novo mesto (SI); ZUPET, Rok, 1000 Ljubljana (SI); SMIRNOVA, Elena, 1000 Ljubljana (SI)
(74) Representative: Hoefer & Partner Patentanwälte mbB
(86) International application number: PCT/EP2014/050411
(87) International publication number: WO 2014/108514

(56) References cited:
- US-A1- 2011 071 120
- CAIRA M R: "CRYSTALLINE POLYMORPHISM OF ORGANIC COMPOUNDS", TOPICS IN CURRENT CHEMISTRY, SPRINGER, BERLIN, DE, vol. 198, 1 January 1998 (1998-01-01), pages 163-208, XP001156954, ISSN: 0340-1022, DOI: 10.1007/3-540-69178-2_5
- None

## Description

### FIELD OF THE INVENTION

Disclosed herein are polymorphic forms of tapentadol maleate and uses thereof, as well as a process for the preparation of crystalline tapentadol maleate, a process for the preparation of tapentadol maleate Form II from tapentadol maleate Form I or enriching mixtures of tapentadol maleate Form I and II, and a process for the preparation of tapentadol maleate Form I or II or mixtures thereof.

### BACKGROUND OF THE INVENTION

Tapentadol of formula I with its chemical name 3-((1R,2R)-3-(dimethylamino)-1-ethyl-2-methylpropyl)phenol or alternatively 3-((2R,3R)-1-(dimethylamino)-2-methylpentan-3-yl)phenol, is marketed under the trade name Nucynta and indicated for the treatment of moderate to severe pain. 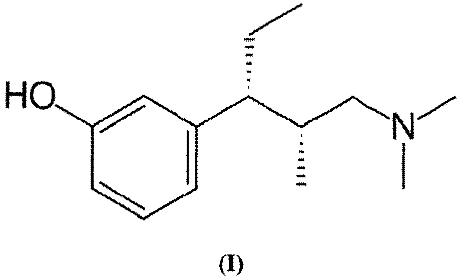 Tapentadol hydrochloride, its synthesis, and its use as an analgesic are disclosed in European patent EP0693475 B1. Various processes for the preparation of tapentadol, its enantiomers and related compounds, and their pharmaceutically acceptable salts are disclosed in EP0693475 B1; and PCT patent applications WO 2004/108658, WO 2005/000788, WO 2008/012046, WO 2008/012047, and WO 2008/012283. The basic patent for tapentadol, EP0693475 B1, discloses processes for the preparation of tapentadol or a pharmaceutically acceptable salt thereof. While it mentions that some of the disclosed compounds can Form salts with physiologically acceptable acids, for example, hydrochloric acid, hydrobromic acid, sulfuric acid, methanesulfonic acid, formic acid, acetic acid, oxalic acid, succinic acid, tartaric acid, mandelic acid, fumaric acid, lactic acid, citric acid, glutamic acid and/or aspartic acid, only the hydrochloride salt of tapentadol had been prepared and isolated.

European patents EP1612203 and EP1799633 disclose polymorphic Forms A and B of tapentadol hydrochloride, and characterize them by powder X-ray diffraction (P-XRD), Infra Red spectroscopy (IR), RAMAN spectroscopy and crystal structure analysis. EP1612203 further teaches that the procedure described in example 25 of U.S. Pat. No. 6,248,737 and U.S. Pat. No. 6,344,558 as well as EP 693475 B1 produces crystalline Form B of tapentadol hydrochloride.

US2011071120 claims novel solid state Forms of tapentadol salts, process for their preparation, pharmaceutical compositions, and method of treating thereof. The tapentadol salts include an L-(-)-camphorsulfonate salt, a dibenzoyl-(L)-tartrate salt, a dibenzoyl-(D)-tartrate salt, a malate salt, a maleate salt, or a salicylate salt. In example 5, the isolation of tapentadol maleate is presumably disclosed.

WO2012010316 relates to a salt or cocrystal of 3-(3-dimethylamino-1-ethyl-2-methyl-propyl)-phenol (component a) and at least one acid component (b1) or at least one acid component (b2), wherein the salt or cocrystal of component (a) and component (b2) is present in crystalline and/or amorphous Form, a medicament comprising said salt or cocrystal as well as said salt or cocrystal for use in the treatment of pain. Only the preparation and isolation of tapentadol salts/co-crystals with (2S,3S)-dibenzoyl tartaric acid, sebacic acid, 1-hydroxy-2-naphthoic acid, embonic acid, nitric acid, nicotinic acid, hydrobromic acid, sulfuric acid, fumaric and malonic acid is disclosed in the examples.

WO2012051246 discloses tapentadol hydrobromide, and crystalline Forms thereof. Example 8 of WO2012051246 states the phosphate salt to become liquid a few seconds after removal of solvent and that the sulfuric salt of tapentadol could not be isolated at all. The reaction of tapentadol free base with the other acids, among other maleic acids, is reported to have resulted in a bitumen and no crystalline salt form could be isolated.

There remains a need for novel salts and novel solid state forms of tapentadol salts.

### SUMMARY OF THE INVENTION

In one aspect, provided herein are polymorphic forms of tapentadol maleate and their use in pharmaceutical dosage forms. Provided herein is crystalline tapentadol maleate, said crystalline tapentadol maleate being crystalline tapentadol maleate characterized by the following 2-theta degrees: 11,5; 18,6; 22,1 ±0.2, or crystalline tapentadol maleate characterized by the following 2-theta degrees: 17,1; 19,2; 19,9 ±0.2, in particular, said crystalline tapentadol maleate having a water content from 0 to 5 w/w %, preferably 0.02 to 4.9 w/w %, most preferably 0.1 to 4 w/w %. Said crystalline tapentadol maleate can furthermore be a hydrated or anhydrous crystalline form of tapentadol maleate. Provided herein is also crystalline tapentadol maleate hemihydrate, characterized by the following 2-theta degrees: 17,1; 19,2; 19,9 ±0.2.

Described herein is a process for preparing a crystalline or amorphous form, hydrated and anhydro forms of tapentadol maleate, tapentadol maleate with a water content of 0 to 5 w/w%, comprising contacting tapentadol free base with maleic acid in a suitable solvent under suitable conditions to produce a reaction mass, and isolating the solid state form of tapentadol maleate.

In another aspect, provided herein is a mixture of tapentadol maleate comprising Form I and/or Form II tapentadol maleate, tapentadol maleate Form I being characterized by the following 2-theta degrees: 11,5; 18,6; 22,1 ±0.2, and tapentadol maleate Form II being characterized by the following 2-theta degrees: 17,1; 19,2; 19,9 ±0.2.

In another aspect, provided herein is a mixture of tapentadol maleate, said mixture being a mixture of anhydrous and hydrated crystalline forms of tapentadol maleate, anhydrous form being Form I of tapentadol maleate, tapentadol maleate Form I being characterized by the following 2-theta degrees: 11,5; 18,6; 22,1 ±0.2

In another aspect, provided herein is a process for the preparation of crystalline tapentadol maleate comprising the following steps:
i. dissolving tapentadol free base in a first solvent, the first solvent being selected from (C1-C6) alcohols, nitriles, halogenated hydrocarbons, cyclic halogenated or non-halogenated hydrocarbons, esters, ketones, dimethylsulfoxide (DMSO), dimethylformamide (DMF) and mixtures thereof,
ii. addition of maleic acid for the formation of tapentadol maleate solution,
iii. evaporation of the first solvent until minimal concentration of 500 mg tapentadol maleate per g of solution is reached,
iv. addition of a second solvent to the obtained residue after evaporation,
v. mixing the crystallization mixture at crystallization temperature until a suspension is formed,
vi. isolation and drying of the formed solid.

In another aspect, provided herein is a process for the preparation of tapentadol maleate Form II from tapentadol maleate Form I or enriching mixtures of tapentadol maleate Form I and II by applying mechanical stress to tapentadol maleate, in particular by applying mechanical stress to tapentadol maleate Form I or to a mixture comprising or consisting of tapentadol maleate Form I and II, tapentadol maleate Form I being characterized by the following 2-theta degrees: 11,5; 18,6; 22,1 ±0.2, and tapentadol maleate Form II being characterized by the following 2-theta degrees: 17,1; 19,2; 19,9 ±0.2. The mechanical stress can be applied by grinding.

In another aspect, provided herein is a process for the preparation of tapentadol maleate Form I or II or mixtures thereof by seeding the solution of tapentadol maleate with crystals of tapentadol maleate Form I or II or mixtures thereof,
tapentadol maleate Form I being characterized by the following 2-theta degrees: 11,5; 18,6; 22,1 ±0.2, and tapentadol maleate Form II being characterized by the following 2-theta degrees: 17,1; 19,2; 19,9 ±0.2.

In another aspect, provided herein is a use of crystalline tapentadol maleate, characterized in water content from 0 - 5 w/w % for the preparation of pharmaceutical dosage forms, said crystalline tapentadol maleate being selected from tapentadol maleate Form I and tapentadol maleate Form II, tapentadol maleate Form I being characterized by the following 2-theta degrees: 11,5; 18,6; 22,1 ±0.2, and tapentadol maleate Form II being characterized by the following 2-theta degrees: 17,1; 19,2; 19,9 ±0.2.

Described herein is a method for treating a patient suffering from severe acute pain comprising administering a solid state form of tapentadol maleate along with pharmaceutically acceptable excipients.

Described herein is a pharmaceutical composition comprising a solid state form of tapentadol maleate as disclosed or described herein, and one or more pharmaceutically acceptable excipients.

Described herein is a pharmaceutical composition comprising a solid state form of tapentadol maleate made by the process disclosed herein, and one or more pharmaceutically acceptable excipients.

Described herein is a process for preparing a pharmaceutical formulation comprising combining any one of the solid state forms of tapentadol maleate disclosed herein with one or more pharmaceutically acceptable excipients.

In another aspect, the solid state forms of tapentadol maleate, said solid state forms of tapentadol maleate being crystalline Form I of tapentadol maleate or crystalline Form II of tapentadol maleate, are disclosed herein for use in the pharmaceutical compositions having particle size determined by microscopic method in the range of 1 to 500 µm preferably 5 to 300 µm.

Described herein is a process for preparing tapentadol free base comprising deprotection of an *O-*protected intermediate in salt form and conversion of the tapentadol salt into the free base form.

Described herein is also a process for preparing tapentadol free base comprising deprotection of an O-protected intermediate in the free base form.

Tapentadol maleate and its polymorphic forms of the present invention may be prepared directly from the isolated tapentadol base, from a reaction mixture comprising tapentadol base or by converting any tapentadol salt into its free base followed by its conversion into the maleate salt.

### BRIEF DESCRIPTION OF THE DRAWINGS

FIG. 1 is a characteristic Powder X-ray Diffraction (XRD) pattern of crystalline Form I of tapentadol maleate.
FIG. 2 is a characteristic Powder X-ray Diffraction (XRD) pattern of crystalline Form II of tapentadol maleate.

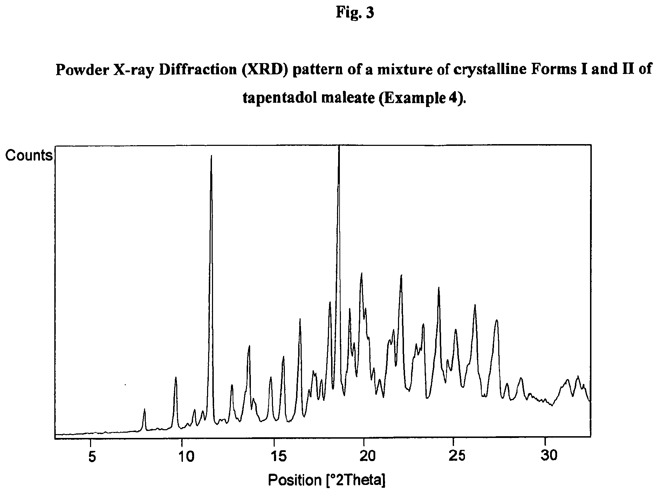

FIG. 3 is a characteristic Powder X-ray Diffraction (XRD) pattern of a mixture of crystalline Forms I and II of tapentadol maleate.

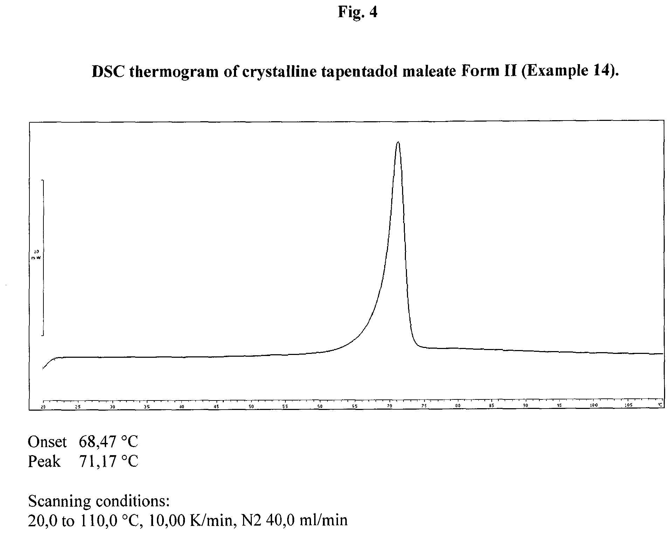

Fig. 4 is a DSC thermogram of crystalline tapentadol maleate Form II (Example 14).

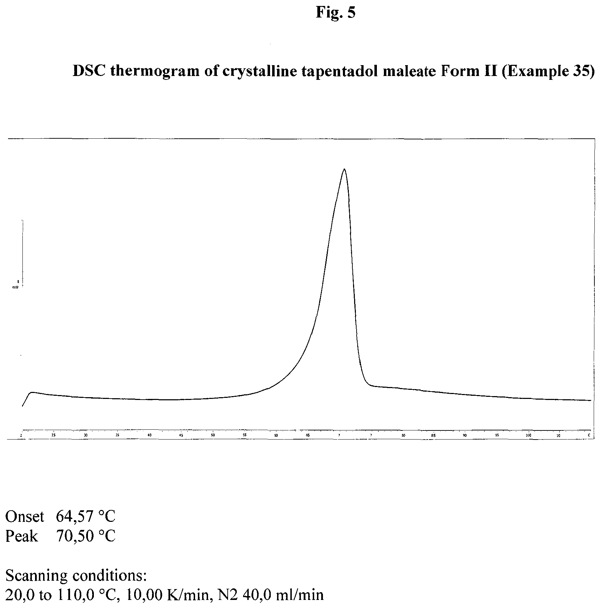

Fig. 5 is a DSC thermogram of crystalline tapentadol maleate Form II (Example 35).

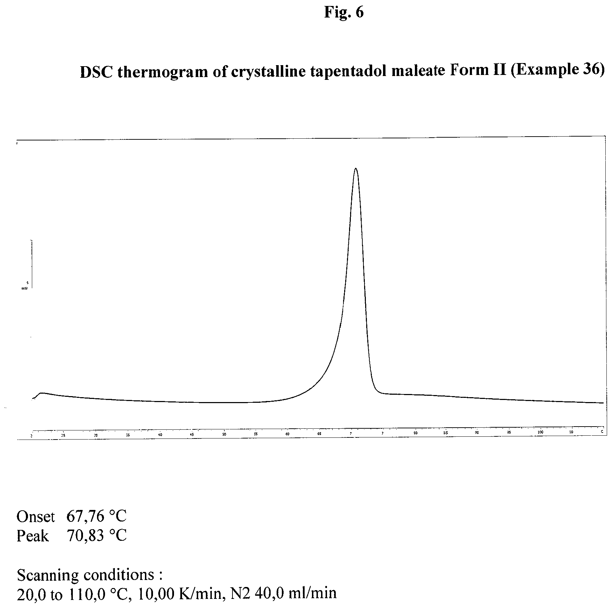

Fig. 6 is a DSC thermogram of crystalline tapentadol maleate Form II (Example 36).

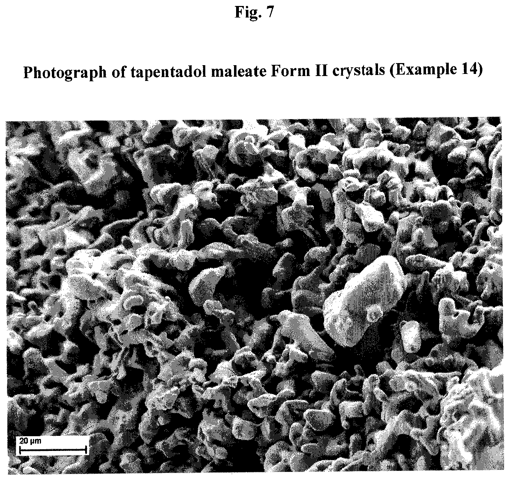

Fig. 7 is a photograph of tapentadol maleate Form II crystals (Example 14).

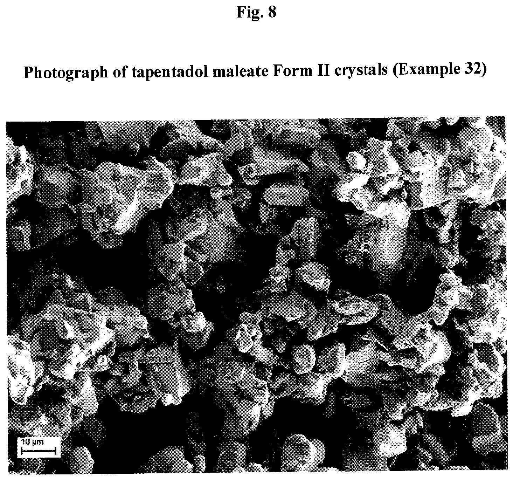

Fig. 8 is a photograph of tapentadol maleate Form II crystals (Example 32).

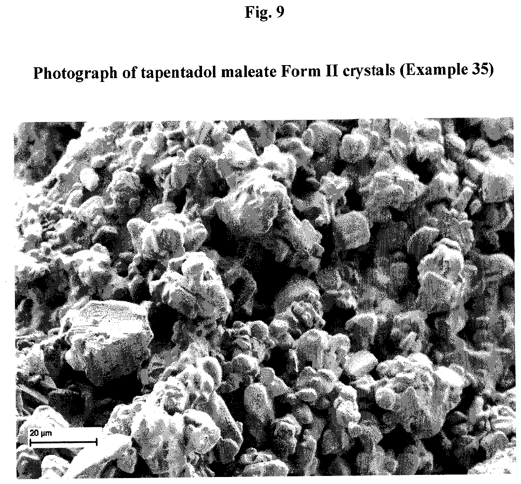

Fig. 9 is a photograph of tapentadol maleate Form II crystals (Example 35).

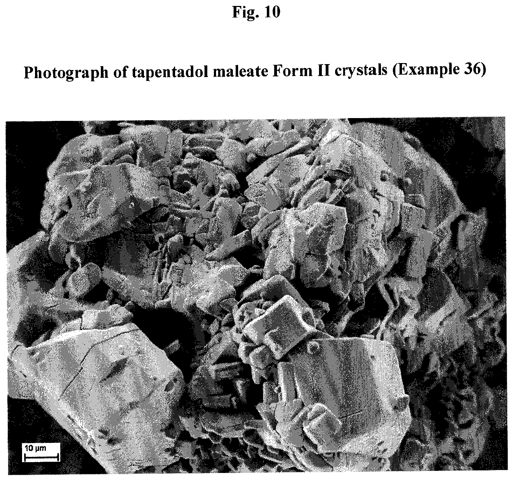

Fig. 10 is a photograph of tapentadol maleate Form II crystals (Example 36).

### DETAILED DESCRIPTION OF THE INVENTION

Crystalline tapentadol maleate and various polymorphic forms thereof were produced according to the following manufacturing method(s).

Crystalline tapentadol maleate, can be produced by a process comprising the following steps:
i) dissolving tapentadol free base in a first solvent,
   the first solvent being selected from (C1-C6) alcohols, nitriles, halogenated hydrocarbons, cyclic halogenated or non-halogenated hydrocarbons, esters, ketones, dimethylsulfoxide (DMSO), dimethylformamide (DMF) and mixtures thereof,
ii) addition of maleic acid for Formation of tapentadol maleate solution, in particular addition of maleic acid to the solution resulting from step (i),
iii) evaporation of the first solvent until minimal concentration of 500 mg tapentadol maleate per g of solution is reached,
iv) addition of a second solvent to the obtained residue after evaporation,
v) mixing the crystallization mixture, in particular the crystallization mixture obtained after step iv), at crystallization temperature until a suspension is formed,
vi) isolation and drying of the Formed solid, in particular isolation of the formed solid from the suspension prepared in step v) and drying of the formed solid after isolation,
vii) optionally aging the dried product in an atmosphere comprising water, preferably air.

Thus, according to this aspect of the present invention, crystalline tapentadol maleate can be obtained by the above process including isolation and drying of the formed solid comprising or consisting of crystalline tapentadol maleate according to step vi) or vii).

As already mentioned above, tapentadol maleate may also be prepared by using a reaction mixture or diluted reaction mixture comprising tapentadol maleate in step i.). In step i.), tapentadol free base can be comprised in a reaction mixture or diluted reaction mixture.

The molar ratio between maleic acid and tapentadol added may be between 0.25 to 2, preferably between 0.5 to 1.5, most preferably 0.7 to 1.3. Maleic acid can be added either in solid or in dissolved form. In particular, in step ii) maleic acid can be added in a molar ratio (maleic acid to tapentadol free base) between 0.25 to 2, preferably between 0.5 to 1.5, most preferably 0.7 to 1.3.

In step iii) the solvent is evaporated until the minimal concentration 500 mg of tapentadol maleate per g of solution is reached, preferably the concentration reached is higher than 600 mg/g, more preferably higher than 700 mg/g.

In step iv) after addition of the second solvent the concentration of tapentadol maleate in crystallization mixture is between 10 and 500 g/L, preferably between 20 and 200 g/l, more preferably between 40 and 120 g/L.

Crystallization temperature, which according to the present invention means the temperature at which the formation of crystals of the product takes place, can be between -20 °C and the reflux temperature (boiling point) of the reaction mixture, preferably between 0°C to 50 °C, most preferably between 10 °C and 30 °C.

In case of remaining insoluble material in stage i) the same or different solvent(s) are added, or the mixture is heated to a temperature between room temperature and the boiling point of the solution until all solid phase is dissolved and remains dissolved upon cooling. When insoluble material is still present in spite of dilution and/or heating, the mixture can be filtered in order to remove it before crystallization or precipitation of the salt. Normally, cooling the mixture more rapidly results in smaller particles compared to when cooling it gradually. In case higher purity is desirable, the person skilled in the art may employ any of the conventional manners of recrystallization.

The resulting crystals or precipitates are usually collected by filtration or centrifugation and washed with a suitable solvent.

Suitable first solvents to be used in step i) and ii) i.e. in the phase of formation of tapentadol maleate can be selected from (C1-C6) alcohols, nitriles such as acetonitrile, halogenated hydrocarbons such as dichloromethane and chloroform, cyclic halogenated or non-halogenated hydrocarbons, esters, ketones, dimethylsulfoxide (DMSO), dimethylformamide (DMF) and mixtures thereof. Suitable second solvents that can be used in step iv) can be selected from cyclic or acyclic liquid hydrocarbons such as *n*-hexane, *n*-heptane and cyclohexane; ethers such as methyl tert-butyl ether, 2-methyltetrahydrofuran, cyclopentyl methyl ether, diisopropyl ether; esters such as isopropyl acetate, butyl acetate, and mixtures thereof. In one embodiment, the first solvent is selected from the group consisting of methanol, ethanol, n-propanol, isopropyl alcohol, isobutanol, n-butanol, tert-butanol, acetone, methyl ethyl ketone, methyl isobutyl ketone, methyl tert-butyl ketone, methylene chloride, ethylene dichloride, chloroform, n-pentane, n-hexane, n-heptane, cyclohexane, toluene, xylene, acetic acid, acetonitrile, N,N-dimethylformamide, N,N-dimethylacetamide, dimethylsulfoxide, and mixtures thereof. Preferably, the first solvent is selected from the group consisting of methanol, ethanol, isopropyl alcohol, acetone, dichloromethane, chloroform and mixtures thereof. The second solvent is selected preferably from the group consisting of methyl tert-butyl ether, n-heptane, 1,4-dioxane, *n*-hexane, butyl acetate and diisopropyl ether and mixtures thereof.

As used herein, "reflux temperature" means the temperature at which the solvent or solvent system refluxes or boils at atmospheric pressure.

Tapentadol free base (solid form, oily form or solution in the specified solvents, in a reaction mixture or in a diluted reaction mixture) as used in the preparation of tapentadol maleate of the present invention may be produced according to any of the reported manufacturing method, or any method employed by the person skilled in the art.

A suitable method comprises deprotection of an *O*-protected intermediate, such as (2*R*,3*R*)-3-(3-(benzyloxy)phenyl)-*N,N*,2-trimethylpentan-1-amine, in salt form and conversion of the tapentadol salt thus obtained into the free base form. Another suitable method comprises deprotection of an *O*-protected intermediate, such as (2*R*,3*R*)-3-(3-(benzyloxy)phenyl)-*N*,*N*,2-trimethylpentan-1-amine, in the free base form.

In case tapentadol base of increased purity is desired, the preparation of tapentadol maleate may also be used as a purification step, and the tapentadol maleate then converted to tapentadol base or another tapentadol salt.

The isolated material containing tapentadol maleate is dried in a state of the art dryer such as a fluid bed dryer, a tray dryer with or without vacuum, or a rotating dryer (application of air flow). In order to facilitate elimination of residual organic solvent one can use a fluid bed dryer where the inlet air has water content in the range of 0.5 to 10 g per kg of air. In some cases higher humidity of inlet air is desired in order to prevent the fast drying of the surface of the material in the dryer resulting in forming a strongly impermeable layer on the surface of the particles causing slow and incomplete removal of solvent from the interior of the particles. Also, after the removal of organic solvent is completed, the as-prepared material can be left to stand in air (or applied airflow over the solid product tapentadol maleate), with water content between 0,01 to 10 g per kg of air, until anhydrous forms present in the material are spontaneously transformed to stable, hydrated form of tapentadol maleate (water content between 0 and 5 w/w %, preferably 0.02 to 4.9 w/w %, most preferably 0.1 to 4 w/w %). Anhydrous form can be Form I or any other anhydrous crystalline form of tapentadol maleate.

The obtained tapentadol maleate has a particle size determined by microscopic method in the range of 1 to 500 µm, preferably 5 to 300 µm. The surface area of such particles can be in the range of 0.1 to 20, preferably 0.5 to 5 m²/g, determined by nitrogen adsorption (BET method). The particle size of tapentadol maleate can be reduced and the particle size distribution can be narrowed, if advantageous for the manufacturing of solid dosage forms, e.g. by conventional milling or grinding methods and equipment such as jet mills and/or hammer mills.

Another aspect of the present invention is the new polymorphic Form I of tapentadol maleate, characterized by the following 2-theta degrees ±0.2 (Table 1):

| No. | Pos. [°2Th.] | Rel. Int. [%] |
|---|---|---|
| 1 | 9,7 | 15 |
| 2 | 11,5 | 93 |
| 3 | 16,4 | 38 |
| 4 | 18,1 | 43 |
| 5 | 18,6 | 100 |
| 6 | 19,8 | 39 |
| 7 | 22,1 | 52 |
| 8 | 23,3 | 35 |
| 9 | 26,1 | 32 |
| **Table 1.** | | |

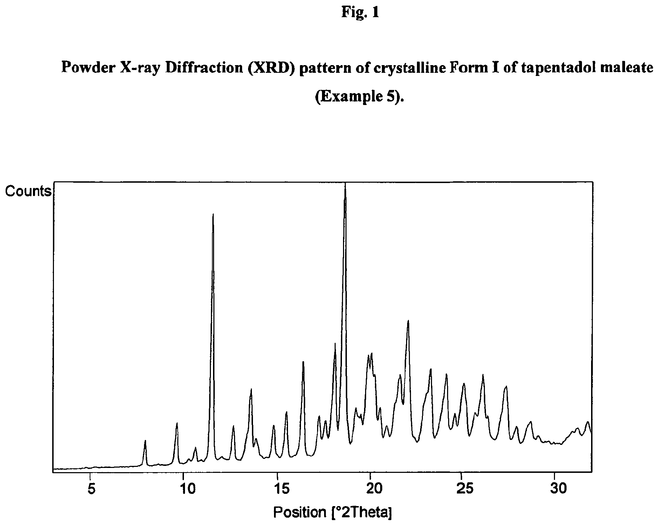

Polymorphic Form I of tapentadol maleate is further characterized by a Powder X-ray Diffraction (XRD) pattern substantially in accordance with Fig. 1. Form I can be prepared according to the process for preparation of tapentadol maleate according to the present invention.

Crystalline tapentadol maleate Form I, in particular crystalline tapentadol maleate Form I having a water content from 0 to 5 w/w %, especially hydrated or anhydrous crystalline tapentadol maleate Form I, can be characterized by the following 2-theta degrees: 11,5; 18,6; 22,1 ±0.2, in particular can be further characterized by the following 2-theta degrees: 11,5; 16,4; 18,1; 18,6; 19,8; 22,1 ±0.2, especially can be further characterized by the following 2-theta degrees: 9,7; 11,5; 16,4; 18,1; 18,6; 19,8; 22,1; 23,3; 26,1 ±0.2.

Another aspect of the invention is polymorphic Form II of tapentadol maleate which is characterized by the following 2-theta degrees ±0.2 (Table 2):

| No. | Pos. [°2Th.] | Rel. Int. [%] |
|---|---|---|
| 1 | 11,1 | 29 |
| 2 | 12,8 | 40 |
| 3 | 16,9 | 56 |
| 4 | 17,1 | 96 |
| 5 | 19,2 | 100 |
| 6 | 19,9 | 83 |
| 7 | 21,4 | 46 |
| 8 | 22,8 | 49 |
| 9 | 24,0 | 49 |
| 10 | 27,3 | 45 |
| **Table 2.** | | |

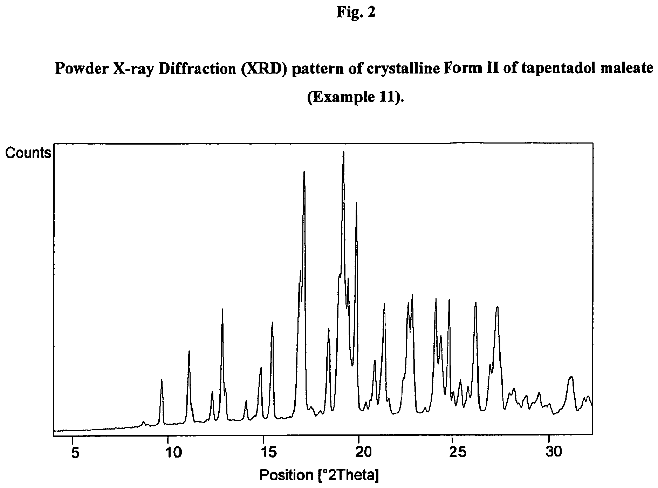

Polymorphic Form II is further characterized by characteristic Powder X-ray Diffraction (XRD) pattern substantially in accordance with Fig. 2.

Crystalline tapentadol maleate Form II, in particular crystalline tapentadol maleate Form II having a water content from 0 to 5 w/w %, especially hydrated or anhydrous crystalline tapentadol maleate Form II, can be characterized by the following 2-theta degrees: 17,1; 19,2; 19,9 ±0.2, in particular can be further characterized by the following 2-theta degrees: 16,9; 17,1; 19,2; 19,9; 22,8; 24,0 ±0.2, especially can be further characterized by the following 2-theta degrees: 16,9; 17,1; 19,2; 19,9; 21,4; 22,8; 24,0; 27,3 ±0.2, especially can be further characterized by the following 2-theta degrees: 11,1; 12,8; 16,9; 17,1; 19,2; 19,9; 21,4; 22,8; 24,0; 27,3 ±0.2.

X-ray powder diffraction patterns were obtained by Phillips PW3040/60 X'Pert PRO diffractometer; CuK_{α} radiation 1,541874 Ǻ.

Provided herein is furthermore a mixture of tapentadol maleate comprising Form I and/or Form II tapentadol maleate, in particular a mixture of tapentadol maleate comprising Form I characterized by the following 2-theta degrees: 11,5; 18,6; 22,1 ±0.2, and/or Form II characterized by the following 2-theta degrees: 17,1; 19,2; 19,9 ±0.2.

Another embodiment of the present invention is directed to a mixture of tapentadol maleate comprising anhydrous or hydrated forms of tapentadol maleate.

According to the presented examples, using the described process of tapentadol maleate salt preparation, following the crystallization, isolation and drying steps results with polymorphic Form I, any mixture of polymorphic Forms I and II, the anhydro form or the hydrated form of tapentadol maleate. Tapentadol maleate comprising 2,5 % w/w of water stoichiometrically corresponds to the hemihydrate form of tapentadol maleate. However, when the prepared material is left to stand at a defined temperature for defined period of time, transformation of polymorphic Form I (or mixture of Form I and II or any other anhydrous form of tapentadol maleate) into polymorphic Form II occurs, resulting in material characterized as pure polymorphic Form II of tapentadol maleate. A typical procedure for the preparation of polymorphic Form II tapentadol maleate is to allow Form I, or mixture of I and II (wet or dry) or any other mixture of anhydrous polymorphic forms or amorphous form of tapentadol maleate, to transform into polymorphic Form II. Polymorphic Form II is prepared when polymorphic Form I is left to stand at a defined temperature and at a defined atmosphere for a defined period of time. The kinetic of transformation into Form II depends on the exact storing conditions: a temperature of between -20 to 75 °C, preferably between 0 and 60 °C, most preferably 15 to 45 °C allows the conversion to take place in 1 hour to 50 days. Applied atmosphere within storing conditions comprises air with relative humidity between 0 and 100 %, preferably between 10 and 80 %, more preferably between 20 and 70 %.

Temperature applied for the transformation of polymorphic Form I (or mixture of Form I and II or any other mixture of anhydrous polymorphic forms or amorphous form of tapentadol maleate) into polymorphic Form II is between -20 and 75 °C, preferably between 0 and 60 °C, most preferably between 15 and 45 °C. The time needed for complete transformation into polymorphic Form II is between 1 hour to 50 days, preferably between 2 hours and 40 days, most preferably between 4 hours and 22 days.

The transformation of polymorphic Form I, or a mixture of polymorphic Forms I and II or any other mixture of anhydrous polymorphic forms or amorphous form of tapentadol maleate, into tapentadol maleate Form II can also be achieved by dispersing the material into a solvent (with or without water) in which of tapentadol maleate shows low solubility and further mixing the formed suspension until the transformation into pure polymorphic Form II is completed. The same effect of polymorphic transition was also observed using air dryer equipment or grinding process for a longer period. Any procedural step applying mechanical stress to tapentadol maleate for a prolonged period of time eventually leads to tapentadol maleate Form II or mixtures of tapentadol maleate Form I and II, in which the content of tapentadol maleate Form II is enriched compared to the content of tapentadol maleate Form II in the initial mixture. The conversion into tapentadol maleate Form II is faster when tapentadol maleate is exposed to air, in particular humid air. When thus drying the isolated tapentadol maleate in step vi.), it is of additional benefit to use a fluid bed dryer where the inlet air has a water content in the range of 0.5 to 10 g per kg of air if the preparation of tapentadol maleate Form II is desired.

Form II or amorphous form of tapentadol maleate can optionally be produced by grinding of the salt sample of Form I or mixture of Form I and II or Form II in a ball mill. Preferably the grinding is performed in the presence of water soluble diluents such as sucrose, lactose in hydrated or anhydrous Form, sugar alcohols such as mannitol, water soluble polymers such as povidone with K value 2 to 50, preferably 7 to 35, cellulose ether such as hypromelose, methyl cellulose, polyvinyl alcohol, graft copolymer of polyvinyl alcohol and polyethyleneglycol, copovidone, polyethyleneglycol or the like, inorganic materials such as colloidal silica (sold under trade name Aerosil), natural silicates such as bentonite or zeolite. During milling the mechanical force exerted on the particle surface leads to particle size reduction, and in the case of tapentadol maleate, also to the conversion to polymorphic Form II of tapentadol maleate. Milling can be performed by any milling process known in the art, for example using a ball mill (planetary ball mill or mixer mill), hammer mill, bead mill, disc mill, ultrasonic mill, torus mill, impact mill, vibration mill, pin mill or air jet mill. The basic principle of treatment in an air jet mill is collision and attrition between particles suspended within the high velocity air stream which introduces the power to the milling chamber. In a ball mill, particles are fractured by impact of grinding media (e.g. balls, cubes, cylinders, jars etc.) that can occupy up to half of the mill chamber volume. Due to rotation of the chamber the grinding media falls from an elevated position. Friction is also present among all elements, contributing significantly to the attrition and consequently to the amorphous nature of the material being milled. One of the most widely used mills in the pharmaceutical industry is the hammer mill. In such equipment, particles are exposed to the impact of rapidly rotating hammers. During milling, material may additionally hit the perforated screen that is placed over the chamber outlet.

Provided herein is a process for the preparation of tapentadol maleate in anhydrous or hydrated crystalline forms. Form I or II or mixtures thereof can be prepared with or without the use of seeding the solution of tapentadol maleate with tapentadol maleate in anhydrous or hydrated crystalline forms. In particular, there is provided a process for the preparation of tapentadol maleate Form I, with or without the use of seeding the solution of tapentadol maleate with crystals of anhydrous or hydrated crystalline forms. In particular, there is provided a process for the preparation of mixture of tapentadol maleate Form I and Form II, with or without the use of seeding the solution of tapentadol maleate with crystals of anhydrous or hydrated crystalline forms. Furthermore, there is provided a process for the preparation of tapentadol maleate Form II, with or without the use of seeding the solution of tapentadol maleate with crystals of anhydrous or hydrated crystalline forms. Also, there is provided a process for the transformation of anhydrous crystalline forms, or mixture of anhydrous and hydrated crystalline forms, into hydrated crystalline form (Form II) by aging the solid material in atmosphere containing water.

Tapentadol maleate of the invention can be used as the drug substance in production of pharmaceutical preparations by combining it with conventional pharmaceutical carriers or diluents employed in this field. The pharmaceutical preparations may be produced by a method usually employed in this field.

The pharmaceutical preparations containing tapentadol maleate of the present invention include orally administrable preparations such as tablets, pills, capsules, granules, powders, liquids and solutions, and the like; or parenteral preparations such as intraarticular, intravenous, or intramuscular injections, suppositories, percutaneous liquid preparations, ointments, transdermal stickers, transmucosal liquid preparations, transmucosal stickers, inhalations, and the like. Particularly, tablets, pills, capsules, granules and powders, are advantageous as stable solid preparations.

In the solid compositions for use in oral administration, one or more of the active ingredients may be mixed with at least one inert diluent, for example, lactose, mannitol, glucose, hydroxypropylcellulose, microcrystalline cellulose, powdered cellulose starch, polyvinylpyrrolidone, magnesium metasilicate aluminate, cyclodextrins and the like. The compositions may contain pharmaceutically acceptable additives other than diluents in a conventional manner, for example, lubricants such as magnesium stearate, sodium stearyl fumarate, hydrogenated castor oil and the like, disintegrating agents such as starch, fibrous calcium glycolate, sodium starch glycolate croscarmellose sodium, crospovidone and the like, stabilizers, or solubilizing agents. The tablets or pills if required may be coated with sugar-coating or a polymeric coating film, such as sucrose, gelatin, hydroxypropylcellulose, hydroxypropylmethylcellulose, and the like. The coating can additionally contain at least one pharmaceutically acceptable additive, selected from colouring agents, opacifiers, antiadherents, and the like.

The methods of preparing the solid oral dosage Forms can be e.g. further be described as:

### a) DIRECT COMPRESSION:

Excipients are mixed in an appropriate mixer such as a biconical mixer. Tapentadol maleate is added and mixed with excipients. To the obtained mixture magnesium stearate is added, homogenous mixture is finally mixed and compressed on tabletting machine. Tablets can be coated with a film coating.

### b) WET GRANULATION:

Excipients with the exception of magnesium stearate are mixed. Tapentadol maleate is added and mixed with excipients and granulated with granulation liquid. To the obtained granulate magnesium stearate is added, homogenous mixture is finally mixed and compressed on tabletting machine. Water, organic solvents miscible with water such as ethanol, isopropanol, methanol, acetone or mixtures thereof can be used as a granulation liquid, in which optional binder or other excipients can be dispersed. Wet granulation can be performed by the state of the art processes and equipment such as low or high shear mixer or fluid bed granulators (top spray, bottom spray or tangential spray). The granulate may be screened and/or milled to obtain a blend with suitable technological properties/sizes. Tablets can be coated with a film coating

### c) DRY GRANULATION

Excipients except magnesium stearate are mixed Tapentadol maleate is added and mixed with excipients. To the obtained mixture a part of magnesium stearate is added. Homogenous mixture is finally mixed and dry granulated via slugging or roller compaction. Obtained slugs or granules can be further milled to achieve the appropriate size of granules. Granules of desirable size are mixed with the rest part of magnesium stearate and compressed into tablets, which can be additionally film coated.

The chemical purity of tapentadol maleate is assessed by high performance liquid chromatography (HPLC) with a column of the type Gemini NX C-18 150 x 4.6 mm i.d., 3 µm particles; column temperature: 25°C;
conditions 1: detector: UV 215 nm; flow rate: 1.0 ml/min; injection volume: 5 µl; mobile phase: A: 0.01M phosphate buffer solution pH 10; B: 90% acetonitrile; Gradient: 0'=25%B, 30'=55%B, 32'-35'=80%B, 37'-41'=25%B.
conditions 2 (applied in Preparative Examples 5 - 7): detector: UV 220 nm; flow rate: 0.8 ml/min; injection volume: 5 µl; mobile phase: A: 0.02M phosphate buffer solution pH 3; B: mixture of 95% acetonitrile and methanol in the ratio 1:1; Gradient: 0'=12%B, 6'=25%B, 9'-15'=40%B, 40'=80%B, 45'-48'=90%B, 52'-37'=12%B.
These HPLC methods are generally applicable for the analysis of tapentadol and its related substances, chromatographic purity and assay.

Enantiomeric purity is assesed by HPLC with a polysaccharide chiral column Chiralpak AD-H, 250 x 4.6 mm, 5 µm particles. For separation is used mobile phase in composition of mixture of heptane, 2-propanol and diethylamine in the ratio 985:15:1 (V/V/V); flow-rate: 1 ml/min; UV detector: 273 nm; injection volume: 5 µl.

### Sample preparation:

Assay and chromatographic purity: The sample solution is prepared in concentration about 1.0 mg/ml. Dilution solvent is 50 % acetonitrile.
Enantiomeric purity: The sample solution is prepared in concentration about 3 mg/ml. The dilution solvent is a mixture of ethanol and hexane (15:85, V/V).

### Calculation:

Chromatographic purity: Use area per cent method. Do not integrate solvent peaks and the maleic acid peak.
Assay: Use external standard method
Enantiomeric purity: Use area per cent method.

In the presented experiments below, all starting materials (Examples 1 and 3) and the products obtained (Example 2, Examples 4-29) are characterized by an enantiomeric purity of more than 99.9 area %.

The invention is explained specifically by the following examples which are not intended as a limitation thereof and are not intended to restrict the scope of the invention.

In the examples "room temperature" is defined between 21 - 24 °C. Also, "laboratory atmosphere" is defined as air with relative humidity between 30 - 60 % and with room temperature (defined above).

"Water content in obtained product" defines the content of water in the obtained products (w/w %) as determined using Karl Fischer titration. Hydrated and anhydro forms of tapentadol maleate may be prepared according to the present invention.

"LOD (120 °C)" defines the amount of water and other volatile matters (w/w %) in a obtained product, determined by drying the sample at the temperature 120 °C.

BET surface may be determined according to DIN 66131. A publication concerning BET surface determination may be also found in J. Am. Chem. Soc. 60, 309 (1938).

Particle size may be in particular determined by microscopically determining the maximum particle diameter of a particle.

All features and embodiments disclosed herein shall be considered as combinable within the present invention, in particular as defined in the claims annexed, and therefore any combination of such features or embodiments disclosed in the present description is part of the present invention, unless explicitly stated otherwise.

### EXAMPLES

### Reference Example 1: Tapentadol free base

Tapentadol hydrochloride (17.0 g, HPLC purity 97.2 area %, HPLC assay 98.6 w/w %), 3-((1R,2R)-3-(dimethylamino)-1-ethyl-2-methylpropyl)phenol hydrochloride, was added to a mixture of dichloromethane (34 mL) and water (34 mL) in a glass vessel equipped with a condenser and magnetic stirrer. The mixture was mixed for the next 10 minutes at the room temperature. Then ammonia solution (25 %) was added drop wise under intense mixing until pH value of 10.0 was reached, then the mixing was stopped. The phases were separated in few minutes and the organic phase 1 was collected. The aqueous phase was further extracted by adding fresh dichloromethane (35 mL) and mixed for 10 minutes. The phases were separated in few minutes and the organic phase 2 was collected. Prepared organic phases (organic phase 1 and 2) were combined and the aqueous phase was discarded. The combined organic phase was transferred to rotary evaporator and the solvent was evaporated using water bath (40 °C) and applied vacuum (final p < 25 mbar). Tapentadol free base, 3-((1R,2R)-3-(dimethylamino)-1-ethyl-2-methylpropyl)phenol, was recovered in form of oil (16.1 g).

### Example 2: Tapentadol maleate (mixture of Form I and Form II)

Tapentadol base prepared in Example 1 (1.0 g, Example 1), 3-((1R,2R)-3-(dimethylamino)-1-ethyl-2-methylpropyl)phenol, was added to dichloromethane (5 mL) in a glass vessel equipped with a condenser and magnetic stirrer. The mixing was applied through the process. The resulting mixture was heated to 39 °C and maintained at this temperature until clear solution was obtained. After that the maleic acid (0.52 g) was added and the mixture was further mixed at the temperature of 39 °C until clear solution was obtained again. The as prepared solution was transferred to rotary evaporator and the solvent was evaporated using water bath (40 °C) and applied vacuum (final p < 25 mbar). The product in form of oil was obtained (1.58 g). Furthermore, methyl *tert*-butyl ether (20 mL, room temperature) was added to obtained product. The mixture was heated to 40 °C and maintained at this temperature for 20 min, and then slowly cooled to room temperature. After the mixture was cooled to room temperature it was further mixed for the next 20 hours. Finally, the formed product was isolated using filtration and washed with 5 mL of methyl tert-butyl ether.

The isolated product was dried in a vacuum drier (28 °C, p < 50 mbar) for the next 24 hours. The product was obtained in the Form of powder (1.1 g, HPLC purity 98.2 area %, HPLC assay 99.0 w/w %, HPLC enantiomeric purity 99.92 area %).

### Reference Example 3: Tapentadol free base

Tapentadol hydrochloride (40.0 g, HPLC purity 99.8 area %, HPLC assay 100.1 w/w %), 3-((1R,2R)-3-(dimethylamino)-1-ethyl-2-methylpropyl)phenol hydrochloride, was added to a mixture of dichloromethane (80 mL) and water (80 mL) in a glass vessel equipped with a condenser and magnetic stirrer. The mixture was mixed for the next 10 minutes at the room temperature. Then ammonia solution (25 %) was added drop wise under intense mixing until pH value of 10.0 was reached, then the mixing was stopped. The phases were separated in few minutes and the organic phase 1 was collected. The aqueous phase was further extracted adding fresh dichloromethane (80 mL) and mixed for 10 minutes. The phases were separated in few minutes and the organic phase 2 was collected. Prepared organic phases (organic phase 1 and 2) were combined and the aqueous phase was discarded. The combined organic phase was transferred to rotary evaporator and the solvent was evaporated using water bath (40 °C) and applied vacuum (final p < 25 mbar). Tapentadol free base, 3-((1R,2R)-3-(dimethylamino)-1-ethyl-2-methylpropyl)phenol, was recovered in the form of oil (37.7 g).

### Example 4: Tapentadol maleate (mixture of Form I and Form II)

Tapentadol base as prepared in Example 3 (10.0 g, Example 3), 3-((1R,2R)-3-(dimethylamino)-1-ethyl-2-methylpropyl)phenol, was added to dichloromethane (50 mL) in a glass vessel equipped with a condenser and magnetic stirrer. The mixing was applied through the process. The resulting mixture was heated to 39 °C and maintained at this temperature until clear solution was obtained. After that the maleic acid (5.24 g) was added and the mixture was further mixed at the temperature of 39 °C until clear solution was obtained again. The as prepared solution was transferred to rotary evaporator and the solvent was evaporated using water bath (40 °C) and applied vacuum (final p < 25 mbar). The product in form of oil was obtained (16.0 g). Furthermore, methyl *tert-butyl* ether (200 mL, room temperature) was added to obtained product. The mixture was heated to 40 °C and maintained at this temperature for 60 min, and then slowly cooled to room temperature. After the mixture was cooled to room temperature it was further mixed for the next 25 hours. Finally, the formed product was isolated using filtration and washed with 10 mL of methyl *tert*-butyl ether.

The isolated product was dried in a vacuum drier (28 °C, p < 50 mbar) for the next 24 hours. The product was obtained in the form of powder (10.9 g, HPLC purity 99.7 area %, HPLC assay 99.1 w/w %, HPLC enantiomeric purity 99.97 area %, water content in obtained product 0.5 %).

### Example 5: Tapentadol maleate Form I

The above prepared tapentadol base (1.0 g, Example 1), 3-((1R,2R)-3-(dimethylamino)-1-ethyl-2-methylpropyl)phenol, was added to dichloromethane (5 mL) in a glass vessel equipped with a condenser and magnetic stirrer. The mixing was applied through the process. The resulting mixture was heated to reflux and maintained at these conditions until clear solution was obtained. After that the maleic acid (0.52 g) was added and the mixture was further mixed at the temperature of reflux until clear solution was obtained again (15 min). The prepared solution was transferred to rotary evaporator and the solvent was evaporated using water bath (40 °C) and applied vacuum (final p < 25 mbar). The product in the form of oil was obtained (1.56 g). Furthermore, methyl tert-butyl ether (10 mL, room temperature) was added to obtained product. The mixture was heated to 40 °C and maintained at this temperature for 20 min, and then slowly cooled to room temperature. After the mixture was cooled to room temperature it was further maintained at this temperature and mixed for the next 72 hours. Finally, the formed product was isolated by filtration and washed with 5 mL of methyl *tert*-butyl ether.

The isolated product was dried in a vacuum drier (25 °C, p < 50 mbar) for the 12 hours. The product was obtained in the Form of powder (1.0 g, HPLC purity 97.8 area %, HPLC assay 98.0 w/w %, HPLC enantiomeric purity 99.95 area %).

### Example 6:

### Example 6a: Tapentadol maleate Form I

Tapentadol base (2.0 g, Example 3), 3-((1R,2R)-3-(dimethylamino)-1-ethyl-2-methylpropyl)phenol, was added to dichloromethane (10 mL) in a glass vessel equipped with a condenser and magnetic stirrer. The mixing was applied through the process. The resulting mixture was heated to 38 °C and maintained at this temperature until clear solution was obtained. After that the maleic acid (1.04 g) was added and the mixture was further mixed at the temperature of 38 °C until clear solution was obtained again (25 min). The prepared solution was transferred to rotary evaporator and the solvent was evaporated using water bath (40 °C) and applied vacuum (final p < 20 mbar). The product in the form of oil was obtained (3.35 g). Furthermore, butyl acetate (40 mL, room temperature) was added to obtained product. The mixture was heated to 40 °C and maintained at this temperature for 60 min, and then slowly cooled to room temperature. After the mixture was cooled to room temperature it was further maintained at this temperature and mixed for the next 72 hours. Finally, the formed product was isolated by filtration.

The isolated product was dried in a vacuum drier (40 °C, p < 50 mbar) for the 6 hours. The product was obtained in the form of powder (2.6 g, HPLC purity 99.7 area %, HPLC assay 99.0 w/w %).

### Example 6b: Tapentadol maleate Form I

Tapentadol base (2.0 g, Example 3), 3-((1R,2R)-3-(dimethylamino)-1-ethyl-2-methylpropyl)phenol, was added to dichloromethane (10 mL) in a glass vessel equipped with a condenser and magnetic stirrer. The mixing was applied through the process. The resulting mixture was heated to 38 °C and maintained at this temperature until clear solution was obtained.

After that the maleic acid (1.05 g) was added and the mixture was further mixed at the temperature of 38 °C until clear solution was obtained again (25 min). The prepared solution was transferred to rotary evaporator and the solvent was evaporated using water bath (40 °C) and applied vacuum (final p < 20 mbar). The product in the form of oil was obtained (3.11 g). Furthermore, butyl acetate (37.5 mL, room temperature) was added to obtained product. The mixture was heated to 60 °C and maintained at this temperature for 35 min, and then slowly cooled to 30 °C. After the mixture was cooled to 30 °C it was further maintained at this temperature and mixed for the next 17 hours. Finally, the formed product was isolated by filtration (under dry nitrogen atmosphere) and washed with 3 mL of butyl acetate.

The isolated product was dried in a vacuum drier (45 °C, p < 50 mbar) for 48 hours. The product was obtained in the form of powder (2.2 g, water content in obtained product 0.2 %, HPLC purity 99.9 area %, HPLC assay 99.6 w/w %, HPLC enantiomeric purity 99.97 area %).

### Example 6c: Tapentadol maleate Form I

Tapentadol base (2.0 g, Example 3), 3-((1R,2R)-3-(dimethylamino)-1-ethyl-2-methylpropyl)phenol, was added to dichloromethane (10 mL) in a glass vessel equipped with a condenser and magnetic stirrer. The mixing was applied through the process. The resulting mixture was heated to 38 °C and maintained at this temperature until clear solution was obtained. After that the maleic acid (1.05 g) was added and the mixture was further mixed at the temperature of 38 °C until clear solution was obtained again (25 min). The prepared solution was transferred to rotary evaporator and the solvent was evaporated using water bath (40 °C) and applied vacuum (final p < 20 mbar). The product in the form of oil was obtained (3.14 g). Furthermore, butyl acetate (37.5 mL, room temperature) was added to obtained product. The mixture was heated to 60 °C and maintained at this temperature for 35 min, and then slowly cooled to room temperature. After the mixture was cooled to room temperature it was further maintained at this temperature and mixed for the next 17 hours. Finally, the formed product was isolated by filtration and washed with 3 mL of butyl acetate.

The isolated product was dried in a vacuum drier (45 °C, p < 50 mbar) for the 48 hours. The product was obtained in the form of powder (2.3 g, water content in obtained product 0.3 %, HPLC purity 99.9 area %, HPLC assay 99.0 w/w %, HPLC enantiomeric purity 99.99 area %).

### Example 7: Tapentadol maleate Form I

Tapentadol base (2.0 g, Example 3), 3-((1R,2R)-3-(dimethylamino)-1-ethyl-2-methylpropyl)phenol, was added to dichloromethane (10 mL) in a glass vessel equipped with a condenser and magnetic stirrer. The mixing was applied through the process. The resulting mixture was heated to 38 °C and maintained at this temperature until clear solution was obtained. After that the maleic acid (1.04 g) was added and the mixture was further mixed at the temperature of 38 °C until clear solution was obtained again (25 min). The prepared solution was transferred to rotary evaporator and the solvent was evaporated using water bath (40 °C) and applied vacuum (final p < 20 mbar). The product in the form of oil was obtained (3.25 g). Furthermore, diisopropyl ether (40 mL, room temperature) was added to obtained product. The mixture was heated to 40 °C and maintained at this temperature for 60 min, and then slowly cooled to room temperature. After the mixture was cooled to room temperature it was further maintained at this temperature and mixed for the next 72 hours. Finally, the formed product was isolated by filtration.

The isolated product was dried in a vacuum drier (40 °C, p < 50 mbar) for the 6 hours. The product was obtained in the form of powder (2.3 g, HPLC purity 99.7 area %, HPLC assay 98.2 w/w %, HPLC enantiomeric purity 99.98 area %).

### Example 8: Tapentadol maleate (mixture of Form I and Form II)

Tapentadol base (2.0 g, Example 3), 3-((1R,2R)-3-(dimethylamino)-1-ethyl-2-methylpropyl)phenol, was added to dichloromethane (10 mL) in a glass vessel equipped with a condenser and magnetic stirrer. The mixing was applied through the process. The resulting mixture was heated to 38 °C and maintained at this temperature until clear solution was obtained. After that the maleic acid (1.04 g) was added and the mixture was further mixed at the temperature of 38 °C until clear solution was obtained again (15 min). The prepared solution was transferred to rotary evaporator and the solvent was evaporated using water bath (40 °C) and applied vacuum (final p < 20 mbar). The product in the form of oil was obtained (3.27 g). Furthermore, *n*-heptane (40 mL, room temperature) was added to the obtained product. The mixture was heated to 40 °C and maintained at this temperature for 60 min, and then slowly cooled to room temperature. After the mixture was cooled to room temperature it was further maintained at this temperature and mixed for the next 72 hours. Finally, the formed product was isolated by filtration.

The isolated product was dried in a vacuum drier (40 °C, p < 50 mbar) for the 6 hours. The product was obtained in the form of powder (2.3 g, HPLC purity 99.6 area %, HPLC assay 96.7 w/w %, LOD(120 °C) 1,9 w/w %, HPLC enantiomeric purity 100.0 area %).

### Example 9: Tapentadol maleate (mixture of Form I and Form II)

Tapentadol base (2.0 g, Example 3), 3-((1R,2R)-3-(dimethylamino)-1-ethyl-2-methylpropyl)phenol, was added to dichloromethane (10 mL) in a glass vessel equipped with a condenser and magnetic stirrer. The mixing was applied through the process. The resulting mixture was heated to 38 °C and maintained at this temperature until clear solution was obtained. After that the maleic acid (1.04 g) was added and the mixture was further mixed at the temperature of 38 °C until clear solution was obtained again (25 min). The prepared solution was transferred to rotary evaporator and the solvent was evaporated using water bath (40 °C) and applied vacuum (final p < 20 mbar). The product in the form of oil was obtained (3.31 g). Furthermore, *n*-hexane (40 mL, room temperature) was added to obtained product. The mixture was heated to 40 °C and maintained at this temperature for 60 min, and then slowly cooled to room temperature. After the mixture was cooled to room temperature it was further maintained at this temperature and mixed for the next 72 hours. Finally, the formed product was isolated by filtration.

The isolated product was dried in a vacuum drier (40 °C, p < 50 mbar) for the 6 hours. The product was obtained in the form of powder (2.4 g, HPLC purity 99.6 area %, HPLC assay 98.3 w/w %, HPLC enantiomeric purity 100.0 area %).

### Example 10: Tapentadol maleate (Form II)

Tapentadol maleate obtained according to Example 2 (0.2 g) was transferred into 10 mL glass tube and closed with plastic cover (in laboratory atmosphere). The material in the tube was left to stand for 15 days at room temperature.

### Example 11: Tapentadol maleate (Form II)

Tapentadol maleate obtained according to Example 2 (0.2 g) was transferred into 10 mL glass tube and closed with plastic cover (in laboratory atmosphere). The material in the tube was left to stand for 20 days at room temperature.

### Example 12: Tapentadol maleate (Form II)

Tapentadol maleate obtained according to Example 4 (0.2 g) was transferred into 10 mL glass tube and closed with plastic cover (in laboratory atmosphere). The material in tube was left to stand for 20 days at room temperature (water content in obtained product 2.1 %).

### Example 13: Tapentadol maleate preparation (Form II)

Material from Example 5 (0.2 g) was transferred into 10 mL glass tube and closed with plastic cover (in laboratory atmosphere). The material in tube was left to stand for the next 20 days at room temperature.

### Example 14: Tapentadol maleate preparation (Form II)

Material from Example 4 (0.2 g) was transferred into 10 mL glass tube and left to stand in laboratory atmosphere. The material in tube was left to stand for the next 20 days at room temperature.

### Example 15: Tapentadol maleate preparation (Form II)

Material from Example 5 (0.2 g) was transferred into 10 mL glass tube and left to stand in laboratory atmosphere. The material in tube was left to stand for the next 20 days at room temperature.

### Example 16: Tapentadol maleate (Form II)

Tapentadol maleate obtained according to Example 4 (0.2 g) was transferred into 100 mL glass tube and closed with plastic cover (in laboratory atmosphere. The material in tube was left to stand for 20 days at room temperature.

### Example 17: Tapentadol maleate preparation (Form II)

Material from Example 5 (0.2 g) was transferred into 100 mL glass tube and closed with plastic cover (in laboratory atmosphere). The material in tube was left to stand for the next 20 days at room temperature.

### Example 18: Tapentadol maleate preparation (Form II)

Material from Example 4 (0.2 g) was transferred into 100 mL glass tube and left to stand open in laboratory atmosphere. The material in tube was left to stand for the next 20 days at room temperature.

### Example 19: Tapentadol maleate preparation (Form II)

Material from Example 5 (0.2 g) was transferred into 100 mL glass tube and left to stand open in laboratory atmosphere. The material in tube was left to stand for the next 20 days at room temperature.

### Example 20: Tapentadol maleate preparation (Form II)

Material from Example 6c (0.2 g) was transferred into 100 mL glass tube and closed with plastic cover (in laboratory atmosphere). The material in tube was left to stand for the next 20 days at room temperature.

Example 21:

### Example 21a: Tapentadol maleate preparation (Form II)

Material from Example 6c (0.2 g) was transferred into 100 mL glass tube and left to stand open in laboratory atmosphere. The material in tube was left to stand for the next 20 days at room temperature.

### Example 21b: Tapentadol maleate preparation (Form II)

Material from Example 6c (0.2 g) was transferred into 100 mL glass tube and left to stand open in laboratory atmosphere. The material in tube was left to stand for the next 3 days at room temperature (water content in obtained product 2.6 %, HPLC purity 99.9 area %, HPLC enantiomeric purity 99.99 area %, HPLC assay 99.3 w/w % on anhydrous substance).

### Example 21c: Tapentadol maleate preparation (Form II)

Material from Example 6c (0.2 g) was transferred into 100 mL glass tube and left to stand open in laboratory atmosphere. The material in tube was left to stand for the next 5 days at room temperature (water content in obtained product 2.5 %, HPLC purity 99.9 area %, HPLC enantiomeric purity 99.99 area %).

### Example 22: Tapentadol maleate preparation (Form II)

Material from Example 7 (0.2 g) was transferred into 100 mL glass tube and closed with plastic cover (in laboratory atmosphere). The material in tube was left to stand for the next 15 days at room temperature.

### Example 23: Tapentadol maleate preparation (Form II)

Material from Example 7 (0.2 g) was transferred into 100 mL glass tube and left to stand open in laboratory atmosphere. The material in tube was left to stand for the next 5 days at room temperature.

### Example 24: Tapentadol maleate preparation (Form II)

Material from Example 8 (0.2 g) was transferred into 100 mL glass tube and closed with plastic cover (in laboratory atmosphere). The material in tube was left to stand for the next 10 days at room temperature.

### Example 25: Tapentadol maleate preparation (Form II)

Material from Example 8 (0.2 g) was transferred into 100 mL glass tube and left to stand open in laboratory atmosphere. The material in tube was left to stand for the next 3 days at room temperature.

### Example 26: Tapentadol maleate preparation (Form II)

Material from Example 9 (0.2 g) was transferred into 100 mL glass tube and closed with plastic cover (in laboratory atmosphere). The material in tube was left to stand for the next 10 days at room temperature.

### Example 27: Tapentadol maleate preparation (Form II)

Material from Example 9 (0.2 g) was mL glass tube and-left to stand open in laboratory atmosphere. The material in tube was left to stand for the next 3 days at room temperature.

### Example 28: Tapentadol maleate preparation (Form II)

Material from Example 6 (0.2 g) was transferred into 100 mL glass tube and left to stand open in laboratory atmosphere. The material in tube was left to stand for the next 3 days at room temperature.

### Example 29: Tapentadol maleate preparation (Form II)

Material from Example 7 (0.2 g) was transferred into 100 mL glass tube and left to stand open in laboratory atmosphere. The material in tube was left to stand for the next 3 days at room temperature.

### Example 30a: Tapentadol maleate tablets composition

| **Strength** | **50mg** | **75mg** | **100mg** |
|---|---|---|---|
| Tapentadol maleate | 76,22 | 114,33 | 152,44 |
| Microcrystalline cellulose | 25,78 | 38,67 | 51,56 |
| Lactose monohydrate | 24 | 36 | 48 |
| Croscarmelose sodium | 6 | 9 | 12 |
| Povidon K30 | 4 | 6 | 8 |
| Mg stearate | 2 | 3 | 4 |
| Opadry II | 4 | 6 | 8 |
| TOTAL | 142 | 213 | 284 |

### Example 30b: Tapentadol maleate tablets composition

| **Strength** | **50mg** | **75mg** | **100mg** |
|---|---|---|---|
| Tapentadol maleate | 76,22 | 114,33 | 152,44 |
| Microcrystalline cellulose | 25,78 | 38,67 | 51,56 |
| Lactose monohydrate | 24 | 36 | 48 |
| Sodium starch glycolate | 6 | 9 | 12 |
| Povidon K30 | 4 | 6 | 8 |
| Mg stearate | 2 | 3 | 4 |
| Opadry II | 4 | 6 | 8 |
| **TOTAL** | 142 | 213 | 284 |

### Example 30c: Tapentadol maleate tablets composition

| **Strength** | **50mg** | **75mg** | **100mg** |
|---|---|---|---|
| Tapentadol maleate | 76,22 | 114,33 | 152,44 |
| Microcrystalline cellulose | 25,78 | 38,67 | 51,56 |
| Lactose monohydrate | 24 | 36 | 48 |
| Crospovidone | 6 | 9 | 12 |
| Povidon K30 | 4 | 6 | 8 |
| Mg stearate | 2 | 3 | 4 |
| Opadry II | 4 | 6 | 8 |
| **TOTAL** | 142 | 213 | 284 |

### Example 30d: Tapentadol maleate tablets composition

| **Strength** | **50mg** | **75mg** | **100mg** |
|---|---|---|---|
| Tapentadol maleate | 76,22 | 114,33 | 152,44 |
| Microcrystalline cellulose | 25,78 | 38,67 | 51,56 |
| Lactose monohydrate | 24 | 36 | 48 |
| Mannitol, Crospovidone and Polyvinyl Acetate, co-processed blend (Ludiflash) | 6 | 9 | 12 |
| Povidon K30 | 4 | 6 | 8 |
| Mg stearate | 2 | 3 | 4 |
| Opadry II | 4 | 6 | 8 |
| **TOTAL** | 142 | 213 | 284 |

### Example 30e: Tapentadol maleate tablets composition

| **Strength** | **50mg** | **75mg** | **100mg** |
|---|---|---|---|
| Tapentadol maleate | 76,22 | 114,33 | 152,44 |
| Microcrystalline cellulose | 25,78 | 38,67 | 51,56 |
| Manitol | 24 | 36 | 48 |
| Croscarmelose sodium | 6 | 9 | 12 |
| Povidon K30 | 4 | 6 | 8 |
| Mg stearate | 2 | 3 | 4 |
| Opadry II | 4 | 6 | 8 |
| **TOTAL** | 142 | 213 | 284 |

### Example 30f: Tapentadol maleate tablets composition

| **Strength** | **50mg** | **75mg** | **100mg** |
|---|---|---|---|
| Tapentadol maleate | 76,22 | 114,33 | 152,44 |
| Calcium hydrogen phosphate | 25,78 | 38,67 | 51,56 |
| Lactose monohydrate | 24 | 36 | 48 |
| Croscarmelose sodium | 6 | 9 | 12 |
| Povidon K30 | 4 | 6 | 8 |
| Mg stearate | 2 | 3 | 4 |
| Opadry II | 4 | 6 | 8 |
| **TOTAL** | 142 | 213 | 284 |

### Example 30g: Tapentadol maleate tablets composition

| **Strength** | **50mg** | **75mg** | **100mg** |
|---|---|---|---|
| Tapentadol maleate | 76,22 | 114,33 | 152,44 |
| Microcrystalline cellulose | 25,78 | 38,67 | 51,56 |
| Lactose monohydrate | 24 | 36 | 48 |
| Croscarmelose sodium | 6 | 9 | 12 |
| Povidon K30 | 4 | 6 | 8 |
| Stearic acid | 2 | 3 | 4 |
| Opadry II | 4 | 6 | 8 |
| **TOTAL** | 142 | 213 | 284 |

### Example 30h: Tapentadol maleate tablets composition

| **Strength** | **50mg** | **75mg** | **100mg** |
|---|---|---|---|
| Tapentadol maleate | 76,22 | 114,33 | 152,44 |
| Microcrystalline cellulose | 25,78 | 38,67 | 51,56 |
| Lactose monohydrate | 24 | 36 | 48 |
| Croscarmelose sodium | 6 | 9 | 12 |
| Povidon K90 | 4 | 6 | 8 |
| Mg stearate | 2 | 3 | 4 |
| Opadry II | 4 | 6 | 8 |
| **TOTAL** | 142 | 213 | 284 |

### Example 30i: Tapentadol maleate tablets composition

| **Strength** | **50mg** | **75mg** | **100mg** |
|---|---|---|---|
| Tapentadol maleate | 76,22 | 114,33 | 152,44 |
| Microcrystalline cellulose | 25,78 | 38,67 | 51,56 |
| Lactose monohydrate | 24 | 36 | 48 |
| Croscarmelose | 6 | 9 | 12 |
| sodium | | | |
| Povidon K30 | 4 | 6 | 8 |
| Mg stearate | 2 | 3 | 4 |
| Pharmacoat 606 | 2 | 3 | 4 |
| Macrogol 4000 | 0,5 | 0,75 | 1 |
| Titanium Dioxide | 0,5 | 0,75 | 1 |
| Talc | 1 | 1,5 | 2 |
| **TOTAL** | 142 | 213 | 284 |

### Example 30i: Tapentadol maleate tablets composition

| **Strength** | **50mg** | **75mg** | **100mg** |
|---|---|---|---|
| Tapentadol maleate | 76,22 | 114,33 | 152,44 |
| Calcium hydrogen phosphate | 25,78 | 38,67 | 51,56 |
| Manitol | 24 | 36 | 48 |
| Crospovidone | 6 | 9 | 12 |
| Povidon K30 | 4 | 6 | 8 |
| Mg stearate | 2 | 3 | 4 |
| Opadry II | 4 | 6 | 8 |
| **TOTAL** | 142 | 213 | 284 |

### Example 31a: Tapentadol maleate Form II

Tapentadol base (2.0 g, Example 3), 3-((1R,2R)-3-(dimethylamino)-1-ethyl-2- was heated to 38 °C and methylpropyl)phenol, was added to dichloromethane (10 mL) in a glass vessel equipped with a condenser and magnetic stirrer. Mixing was applied during the process. The resulting mixture maintained at this temperature until a clear solution was obtained. After that the maleic acid (1.05 g) was added and the mixture was further mixed at the temperature of 38 °C until clear solution was obtained again (25 min). The prepared solution was transferred to rotary evaporator and the solvent was evaporated using water bath (40 °C) and applied vacuum (final p < 20 mbar). The product in the form of oil was obtained (3.33 g). Furthermore, isopropyl acetate (20.0 mL, room temperature) was added to obtained product. The mixture was heated to 60 °C and maintained at this temperature for 25 min and then 0.1 mL of purified water was added. Furthermore, the obtained mixture was slowly cooled to room temperature. After the mixture was cooled to room temperature it was maintained at this temperature and mixed for the next 17 hours, and then further cooled to 10 °C and mixed for additional 3 hours. Finally, the formed product was isolated by filtration and washed with 3 mL of isopropyl acetate.

The isolated product was dried in a vacuum drier (40 °C, p < 50 mbar) for the 48 hours. The product was obtained in the form of powder (2.8 g, water content in obtained product 2.5 %, HPLC purity 99.9 area %, HPLC assay 96.8 w/w %, HPLC enantiomeric purity 100.0 area %).

### Example 31b: Tapentadol maleate Form II

Tapentadol base (2.0 g, Example 3), 3-((1R,2R)-3-(dimethylamino)-1-ethyl-2- was heated to 38 °C and methylpropyl)phenol, was added to dichloromethane (10 mL) in a glass vessel equipped with a condenser and magnetic stirrer. Mixing was applied during the process. The resulting mixture maintained at this temperature until a clear solution was obtained. After that the maleic acid (1.05 g) was added and the mixture was further mixed at the temperature of 38 °C until clear solution was obtained again (25 min). The prepared solution was transferred to rotary evaporator and the solvent was evaporated using water bath (40 °C) and applied vacuum (final p < 20 mbar). The product in the form of oil was obtained (3.33 g). Furthermore, isopropyl acetate (20.0 mL, room temperature) was added to obtained product. The mixture was heated to 60 °C and maintained at this temperature for 25 min and then 0.1 mL of purified water was added. Furthermore, the obtained mixture was slowly cooled to room temperature. After the mixture was cooled to room temperature it was maintained at this temperature and mixed for the next 25 hours, and then further cooled to 10 °C and mixed for additional 3 hours. Finally, the formed product was isolated by filtration and washed with 3 mL of isopropyl acetate.

The isolated product was dried in a vacuum drier (30 °C, p < 50 mbar) for the 10 hours. The product was obtained in the form of powder (2.9 g, water content in obtained product 2.5 %, HPLC purity 99.9 area %, HPLC assay 96.8 w/w %, HPLC enantiomeric purity 100.0 area %).

### Example 32: Tapentadol maleate Form II

Tapentadol base (2.0 g, Example 3), 3-((1R,2R)-3-(dimethylamino)-1-ethyl-2-methylpropyl)phenol, was added to dichloromethane (10 mL) in a glass vessel equipped with a condenser and magnetic stirrer. The mixing was applied through the process. The resulting mixture was heated to 38 °C and maintained at this temperature until clear solution was obtained. After that the maleic acid (1.05 g) was added and the mixture was further mixed at the temperature of 38 °C until clear solution was obtained again (25 min). The prepared solution was transferred to rotary evaporator and the solvent was evaporated using water bath (40 °C) and applied vacuum (final p < 20 mbar). The product in the form of oil was obtained (3.28 g). Furthermore, ethyl acetate (20.0 mL, room temperature) was added to obtained product. The mixture was heated to 60 °C and maintained at this temperature for 25 min and then 0.1 mL of purified water was added. Furthermore, the obtained mixture was slowly cooled to room temperature. After the mixture was cooled to room temperature it was maintained at this temperature and mixed for the next 17 hours and then further cooled to 10 °C. 0.2 g of crystallization seeds (tapentadol maleate material obtained in example 31) was added and mixed for additional 3 hours (10 °C). Finally, the formed product was isolated by filtration and washed with 3 mL of ethyl acetate.

The isolated product was dried in a vacuum drier (40 °C, p < 50 mbar) for the 48 hours. The product was obtained in the form of powder (2.6 g, water content in obtained product 2.3 %, HPLC purity 99.9 area %, HPLC assay 97.9 w/w %, HPLC enantiomeric purity 100.0 area %).

### Example 33: Tapentadol maleate Form II

Tapentadol base (2.0 g, Example 3), 3-((1R,2R)-3-(dimethylamino)-1-ethyl-2-methylpropyl)phenol, was added to dichloromethane (10 mL) in a glass vessel equipped with a condenser and magnetic stirrer. The mixing was applied through the process. The resulting mixture was heated to 38 °C and maintained at this temperature until clear solution was obtained. After that the maleic acid (1.05 g) was added and the mixture was further mixed at the temperature of 38 °C until clear solution was obtained again (25 min). The prepared solution was transferred to rotary evaporator and the solvent was evaporated using water bath (40 °C) and applied vacuum (final p < 20 mbar). The product in the form of oil was obtained (3.30 g). Furthermore, ethyl acetate (37.5 mL, room temperature) was added to obtained product. The mixture was heated to 60 °C and maintained at this temperature for 25 min. Furthermore, the obtained mixture was slowly cooled to room temperature. After the mixture was cooled to room temperature it was maintained at this temperature and mixed for the next 17 hours. Then the mixture was further cooled to 10 °C and 0.2 g of crystallization seeds (tapentadol maleate material obtained in example 31) was added and mixed for additional 3 hours. After that, 0.1 mL of purified water was added and further mixed for additional 3 hours at the temperature 10 °C. Finally, the formed product was isolated by filtration and washed with 3 mL of ethyl acetate. The isolated product was dried in a vacuum drier (40 °C, p < 50 mbar) for the 48 hours. The product was obtained in the form of powder (2.3 g, water content in obtained product 2.6 %, HPLC purity 99.9 area %, HPLC assay 98.6 w/w %, HPLC enantiomeric purity 100.0 area %).

### Example 34: Tapentadol maleate Form II

Tapentadol base (2.0 g, Example 3), 3-((1R,2R)-3-(dimethylamino)-1-ethyl-2-methylpropyl)phenol, was added to dichloromethane (10 mL) in a glass vessel equipped with a condenser and magnetic stirrer. The mixing was applied through the process. The resulting mixture was heated to 38 °C and maintained at this temperature until clear solution was obtained. After that the maleic acid (1.05 g) was added and the mixture was further mixed at the temperature of 38 °C until clear solution was obtained again (25 min). The prepared solution was transferred to rotary evaporator and the solvent was evaporated using water bath (40 °C) and applied vacuum (final p < 20 mbar). The product in the form of oil was obtained (3.25 g). Furthermore, isopropyl acetate (37.5 mL, room temperature) was added to obtained product. The mixture was heated to 60 °C and maintained at this temperature for 25 min. Furthermore, the obtained mixture was slowly cooled to room temperature. After the mixture was cooled to room temperature it was maintained at this temperature and mixed for the next 17 hours. Then the mixture was further cooled to 10 °C and 0.2 g of crystallization seeds (tapentadol maleate material obtained in example 21b) was added and mixed for additional 3 hours. After that, 0.1 mL of purified water was added and further mixed for 3 hours at the temperature 10 °C. Finally, the formed product was isolated by filtration and washed with 3 mL of isopropyl acetate.

The isolated product was dried in a vacuum drier (40 °C, p < 50 mbar) for the 48 hours. The product was obtained in the form of powder (2.7 g, water content in obtained product 2.3 %, HPLC purity 99.8 area %, HPLC enantiomeric purity 99.99 area %).

### Example 35: Tapentadol maleate Form II

Tapentadol base (10.0 g, Example 37), 3-((1R,2R)-3-(dimethylamino)-1-ethyl-2-methylpropyl)phenol, was added to dichloromethane (50 mL) in a glass vessel equipped with a condenser and magnetic stirrer. The mixing was applied through the process. The resulting mixture was heated to 38 °C and maintained at this temperature until clear solution was obtained. After that the maleic acid (5.25 g) was added and the mixture was further mixed at the temperature of 38 °C until clear solution was obtained again (approx. 25 min). The prepared solution was transferred to rotary evaporator and was evaporated using water bath (40 °C) and low pressure until 35.0 g of solution was obtained (approx. 70 % of amount of starting dichloromethane was evaporated). Furthermore, fresh isopropyl acetate (100.0 mL, room temperature) was added to obtained solution and the mixture was evaporated again using water bath (40 °C) and low pressure until 48.6 g of mixture was obtained. Again, another portion of fresh isopropyl acetate (50.0 mL, room temperature) was added to obtained mixture and was evaporated again using water bath (40 °C) and low pressure until 44.0 g of mixture was obtained. The obtained mixture was transfeered to glass reactor (250 mL, FlexyLab-DN60, Systag) equipped with a condenser and mechanical stirrer (paddle type glass mixer) and was heated to 40 °C. At this temperature, 50 mL of pre-heated isopropyl acetate (40 °C) and 0.5 mL of water (room temperature) was added to the mixture in the reactor. Furthermore, the obtained mixture was slowly cooled to 10 °C (cooling rate approx. -0,5 °C/min). After the mixture was cooled to 10 °C, 100 mg of crystallization seeds (tapentadol maleate material obtained in example 32) was added and the obtained mixture was slowly cooled to final crystallization temperature 5 °C. After that, the mixture was further mixed for 5 -10 hours at the temperature 5 °C. Finally, the formed product was isolated by filtration and washed with 10 mL of isopropyl acetate.

The isolated product was dried in a vacuum drier (35 °C, p < 50 mbar) for the 25 hours. The product was obtained in the form of powder (11.4 g, water content in obtained product 2.5 %, HPLC purity 99.7 area %,HPLC assay 99.8 w/w %, HPLC enantiomeric purity 99.99 area %).

### Example 36: Tapentadol maleate Form II

Tapentadol base (10.0 g, Example 37), 3-((1R,2R-)-3-(dimethylamino)-1-ethyl-2-methylpropyl)phenol, was added to dichloromethane (50 mL) in a glass vessel equipped with a condenser and magnetic stirrer. The mixing was applied through the process. The resulting mixture was heated to 38 °C and maintained at this temperature until clear solution was obtained. After that the maleic acid (5.25 g) was added and the mixture was further mixed at the temperature of 38 °C until clear solution was obtained again (approx. 25 min). The prepared solution was transferred to rotary evaporator and was evaporated using water bath (40 °C) and low pressure until 31.5 g of solution was obtained (approx. 75 % of starting dichloromethane was evaporated). Furthermore, fresh isopropyl acetate (100.0 mL, room temperature) was added to obtained solution and the mixture was evaporated again using water bath (40 °C) and low pressure until 48.9 g of mixture was obtained. Again, another portion of fresh isopropyl acetate (50.0 mL, room temperature) was added to obtained mixture and was evaporated again using water bath (40 °C) and low pressure until 44.0 g of mixture was obtained. The obtained mixture was transferred to glass reactor (250 mL, FlexyLab-DN60, Systag) equipped with a condenser and mechanical stirrer (paddle type glass mixer) and was heated to 40 °C. At this temperature, 50 mL of pre-heated isopropyl acetate (40 °C) was added to the mixture in the reactor. Furthermore, the obtained mixture was slowly cooled to 10 °C (cooling rate approx. -0,5 °C/min). After the mixture was cooled to 10 °C, 100 mg of crystallization seeds (tapentadol maleate material obtained in example 21c) was added and the obtained mixture was further mixed at this temperature for the next 35 min. After that, 0,5 mL of water (room temperature) was added to the reactor and the mixture was slowly cooled to 5 °C and further mixed for 25 hours at the temperature 5 °C. Finally, the formed product was isolated by filtration and washed with 10 mL of isopropyl acetate.

The isolated product was dried in a vacuum drier (35 °C, p < 50 mbar) for the 10 hours. The product was obtained in the form of powder (12.0 g, water content in obtained product 2.4 %, HPLC purity 99.7 area %,HPLC assay 98.5 w/w %, HPLC enantiomeric purity 99.96 area %).

### Reference Example 37: Tapentadol free base preparation

The tapentadol free base was prepared using the same procedure and the same batch of starting material (Tapentadol hydrochloride, 40.0 g, HPLC purity 99.8 area %, HPLC assay 100.1 w/w %) as described in the Example 3.

### PREPARATIVE EXAMPLES

### Preparative Example 1: Preparation of (2R3R)-3-(3-(benzyloxy)phenyl)-N,N,2-trimethylpentanamide

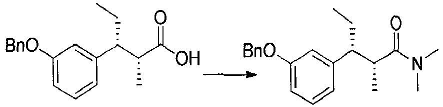

To a stirred solution of (2*R*,3*R*)-3-(3-(benzyloxy)phenyl)-2-methylpentanoic acid (19 g, 0.064 mol,1.0 eq.) in toluene (30 mL) at 25 °C to 35 °C was added dropwise SOCl₂ (9.6 g, 0.081 mol,1.27 eq.) and DMF (0.19 g, 1.0 wt %). The mixture was stirred at 25 °C - 35 °C for 1 h and monitored by TLC. The resulting mixture was added to a suspension of dimethylamine hydrochloride (13.1 g, 0.161 mol, 2.5 eq.) and K₂CO₃ (30.9 g, 0.224 mol, 3.5 eq.) in toluene (30 mL) in a period of 30 min. Then the mixture was heated to 60 °C ∼ 70 °C and stirred for 1 h. The reaction was monitored by HPLC.

After the reaction was completed the reaction mixture was cooled to 10 °C - 20 °C and quenched by the addition of water (40 mL). The two phases were separated and the aqueous phase was extracted with toluene (20 mL). The combined organic phase was washed with 1 M HCl solution (20 mL) and water (20 mL). The organic phase, i.e. the solution of (2*R*,3*R*)-3-(3-(benzyloxy)phenyl)-*N*,*N*,2-trimethylpentanamide in toluene, was used directly in the next step.

### Preparative Example 2: Preparation of (2R,3R)-3-(3-(benzyloxy)phenyl)-N,N,2-trimethylpentan-1-amine hydrochloride

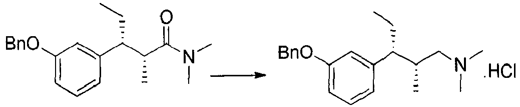

The above toluene solution (from Preparative Example 1) was heated to reflux and traces of water were removed by water separator. Then the solution was cooled to 10 °C-20 °C and Red-Al (∼70 % in toluene, 36.9 g, 0.128 mol, 2.0 eq.) was added dropwise at 10 °C - 20 °C. The resulting mixture was stirred at temperatures of about 30 °C to 40 °C for 1 h and monitored by HPLC.

The reaction mixture was added into the NaOH aqueous solution (1 M, 40 mL) at 0 °C to 5 °C. The two phases were separated and the organic phase was washed twice with water (2 x 20 mL) till pH value of about 7 was reached. Charcoal (1 g) was added, stirred for 0.5 h and filtered off. Purification: The filtrate was acidified with conc. HCl (10 mL) to pH value of 1 to 2. The mixture was heated to reflux and water was removed by water separator. The toluene phase was concentrated under vacuum to reduce volume to about 40 mL. The residue was cooled to 0 °C - 10 °C and stirred for 1 h. The mixture was filtered and dried at 50 °C - 60 °C to afford (*2R,3R*)-3-(3-(benzyloxy)phenyl)-*N*,*N*,2-trimethylpentan-1-amine hydrochloride (20.2 g, 91 % yield for two steps, Preparative Example 1 and 2) as a white solid with a chemical purity of 99 %.

### Preparative Example 3: Preparation of 3-((1R,2R)-3-(dimethylamino)-1-ethyl-2-methylpropyl)phenol hydrochloride (tapentadol hydrochloride)

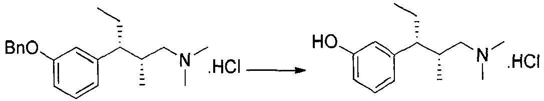

To a stirred solution of (2*R*,3*R*)-3-(3-(benzyloxy)phenyl)-*N*,*N*,2-trimethylpentan-1-amine hydrochloride (20 g, 0.057 mol, from Preparative Example 2) in MeOH (60 mL) was added wet 5 % Pd/C (2 g). The mixture was stirred at 25 °C -35 °C over 8 hrs under hydrogen atmosphere.

The reaction was monitored by HPLC. After the reaction was completed the catalyst was separated and the filtrate was concentrated under vacuum to give 18 g of crude 3-(1*R*,2*R*)-3-(dimethylamino)-1-ethyl-2-methylpropyl)phenol hydrochloride as an oil which was evaporated with *i*-PrOH (2 x 10mL).

Purification: *i*-PrOH (20 mL) was added to the residue and heated to reflux for 1 h, then cooled slowly to 0 °C ∼5 °C. The mixture was stirred at this temperature for 2 hrs, followed by filtration and drying at 50 °C to afford 3-((1*R*,2*R*)-3-(dimethylamino)-1-ethyl-2-methylpropyl)phenol hydrochloride (12.0 g, yield: 81 %) as a white solid.

### Preparative Example 4: Formation and crystallization of 3-((1R,2R)-3-(dimethylamino)-1-ethyl-2-methylpropyl)phenol hydrochloride (tapentadol hydrochloride) from 3-((1R,2R)-3-(dimethylamino)-1-ethyl-2-methylpropyl)phenol (tapentadol free base)

To a stirred solution of 3-((1*R*,2*R*)-3-(dimethylamino)-1-ethyl-2-methylpropyl)phenol_(from Example 3, 2.3 g) in methyl ethyl ketone (33.5 mL) at the temperature 50 °C was added 9.0 mL 1.25 M solution of HCl in isopropyl alcohol (in 10 min). Formed suspension was mixed at 50 °C for the next 60 minutes, followed by slow cooling the crystallization mixture to 30 °C and further mixed for the next 90 minutes. Formed product was isolated using filtration, followed by drying at 30 °C and p < 50 mbar for 24 h. The product was obtained in form of powder (2.25 g, HPLC purity 98.6 area %, HPLC assay 95.7 w/w %).

### Preparative Example 5: Preparation of (2R,3R)-3-(3-(benzyloxy)phenyl)-N,N,2-trimethylpentanamide

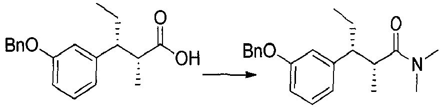

4.86 g of (2*R*,3*R*)-3-(3-(benzyloxy)phenyl)-2-methylpentanoic acid were dissolved in 15 mL of toluene. 1.5 mL of thionyl chloride were added dropwise while stirring under a nitrogen atmosphere. Next, 0.05 mL of DMF were added and the mixture was stirred at about 30 °C for about 70 min.

The reaction mixture was added dropwise to a mixture of 7.81 g of potassium carbonate, 3.29 g of dimethylamine hydrochloride and 10 mL of toluene over a period of 14 min while stirring under a nitrogen atmosphere at about 20 °C. The resulting mixture was stirred at 50 - 55 °C for about 2 h. Next, the mixture was cooled to about 15 - 20 °C and 20 mL of H₂O were added. The phases were separated and the aqueous phase was washed with 7 mL of toluene. The combined organic phases were washed with 7 mL of 2 M HCl and 7 mL of brine, and dried over anhydrous Na₂SO₄.

The mixture was filtered and partially evaporated under reduced pressure. The resulting solution was further diluted with toluene to a volume of 13.5 mL (HPLC purity 99.0 area %).

### Preparative Example 6: Preparation of (2R,3R)-3-(3-(benzyloxy)phenyl)-N,N,2-trimethylpentan-1-amine

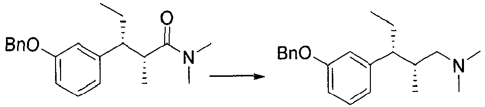

8.9 g of a 70 % solution of sodium bis(2-methoxyethoxy)aluminium dihydride (Red-Al) in toluene were added dropwise over a period of 19 min to 13 mL of the (2R,3R)-3-(3-(benzyloxy)phenyl)-*N*,*N*,2-trimethylpentanamide solution in toluene, prepared in Preparative Example 5, while stirring under a nitrogen atmosphere at about 20 °C. After the addition was completed, the resulting mixture was stirred at about 40 °C under a nitrogen atmosphere for about 2 h. Next, the mixture was added dropwise to 8,1 mL of 2 M NaOH over a period of 22 min while stirring slightly below 10 °C. The phases were separated and the organic phase was washed with 7 x 13 mL of H₂O. To the organic phase, 0.31 g of activated charcoal were added, stirred for 30 min and filtered off. The filtrate was evaporated under reduced pressure to afford 2.95 g of crude (2*R*,3*R*)-3-(3-(benzyloxy)phenyl)-*N*,*N*,2-trimethylpentan-1-amine (HPLC purity 99.2 area %).

### Preparative Example 7: Preparation of 3-((1R,2R)-3-(dimethylamino)-1-ethyl-2-methylpropyl)phenol (tapentadol free base)

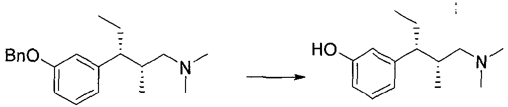

To a solution of (2*R*,3*R*)-3-(3-(benzyloxy)phenyl)-*N*,*N*,2-trimethylpentan-1-amine (4.6 g) in MeOH (34 mL) was added 5 % Pd/C (0.18 g). The mixture was hydrogenated at RT for about 7 h at 150 - 200 mbar hydrogen gauge pressure. After the catalyst was separated, the filtrate was concentrated under reduced pressure to give 2.7 g of crude 3-((1*R*,2*R*)-3-(dimethylamino)-1-ethyl-2-methylpropyl)phenol (tapentadol free base) (HPLC purity 98.5 area %).

## Claims

1. Crystalline tapentadol maleate, said crystalline tapentadol maleate being crystalline tapentadol maleate **characterized by** the following 2-theta degrees: 11,5; 18,6; 22,1 ±0.2 , preferably
further **characterized by** the following 2-theta degrees: 11,5; 16,4; 18,1; 18,6; 19,8; 22,1 ±0.2, more preferably
further **characterized by** the following 2-theta degrees: 9,7; 11,5; 16,4; 18,1; 18,6; 19,8; 22,1; 23,3; 26,1 ±0.2 , or
crystalline tapentadol maleate **characterized by** the following 2-theta degrees: 17,1; 19,2; 19,9 ±0.2, preferably
**characterized by** the following 2-theta degrees: 16,9; 17,1; 19,2; 19,9; 22,8; 24,0 ±0.2, more preferably
further **characterized by** the following 2-theta degrees: 16,9; 17,1; 19,2; 19,9; 21,4; 22,8; 24,0; 27,3 ±0.2, especially
further **characterized by** the following 2-theta degrees: 11,1; 12,8; 16,9; 17,1; 19,2; 19,9; 21,4; 22,8; 24,0; 27,3 ±0.2.

2. Crystalline tapentadol maleate according to claim 1, **characterized in** water content from 0 to 5 w/w %.

3. Crystalline tapentadol maleate according to claim 1, **characterized in that** it is a hydrated or anhydrous crystalline form of tapentadol maleate.

4. Crystalline tapentadol maleate according to claim 1, **characterized in that** it is crystalline tapentadol maleate **characterized by** the following 2-theta degrees: 17,1; 19,2; 19,9 ±0.2, preferably
**characterized by** the following 2-theta degrees: 16,9; 17,1; 19,2; 19,9; 22,8; 24,0 ±0.2, more preferably
further **characterized by** the following 2-theta degrees: 16,9; 17,1; 19,2; 19,9; 21,4; 22,8; 24,0; 27,3 ±0.2, especially
further **characterized by** the following 2-theta degrees: 11,1; 12,8; 16,9; 17,1; 19,2; 19,9; 21,4; 22,8; 24,0; 27,3 ±0.2.

5. Crystalline tapentadol maleate according to claim 4, which is crystalline tapentadol maleate hemihydrate.

6. Crystalline tapentadol maleate according to claim 1, **characterized in that** it is crystalline tapentadol maleate **characterized by** the following 2-theta degrees: 11,5; 18,6; 22,1 ±0.2 , preferably
further **characterized by** the following 2-theta degrees: 11,5; 16,4; 18,1; 18,6; 19,8; 22,1 ±0.2, more preferably
further **characterized by** the following 2-theta degrees: 9,7; 11,5; 16,4; 18,1; 18,6; 19,8; 22,1; 23,3; 26,1 ±0.2.

7. A mixture of tapentadol maleate comprising Form I and/or Form II tapentadol maleate, tapentadol maleate Form I being **characterized by** the following 2-theta degrees: 11,5; 18,6; 22,1 ±0.2, and
tapentadol maleate Form II being **characterized by** the following 2-theta degrees: 17,1; 19,2; 19,9 ±0.2.

8. A mixture of tapentadol maleate, said mixture being a mixture of anhydrous and hydrated crystalline forms of tapentadol maleate, anhydrous form being Form I of tapentadol maleate, tapentadol maleate Form I being **characterized by** the following 2-theta degrees: 11,5; 18,6; 22,1 ±0.2.

9. A process for the preparation of crystalline tapentadol maleate comprising the following steps:
i. dissolving tapentadol free base in a first solvent, the first solvent being selected from (C1-C6) alcohols, nitriles, halogenated hydrocarbons, cyclic halogenated or non-halogenated hydrocarbons, esters, ketones, dimethylsulfoxide (DMSO), dimethylformamide (DMF) and mixtures thereof,
ii. addition of maleic acid for the formation of tapentadol maleate solution,
iii. evaporation of the first solvent until minimal concentration of 500 mg tapentadol maleate per g of solution is reached,
iv. addition of a second solvent to the obtained residue after evaporation,
v. mixing the crystallization mixture at crystallization temperature until a suspension is formed,
vi. isolation and drying of the formed solid.

10. A process for the preparation of tapentadol maleate Form II from tapentadol maleate Form I or enriching mixtures of tapentadol maleate Form I and II by applying mechanical stress to tapentadol maleate, in particular by applying mechanical stress to tapentadol maleate Form I or to a mixture comprising or consisting of tapentadol maleate Form I and II,
tapentadol maleate Form I being **characterized by** the following 2-theta degrees: 11,5; 18,6; 22,1 ±0.2, and
tapentadol maleate Form II being **characterized by** the following 2-theta degrees: 17,1; 19,2; 19,9 ±0.2.

11. A process according to claim 10, wherein the mechanical stress is applied by grinding.

12. A process according to claim 9, wherein in step vi.) the drying of tapentadol maleate isolated is performed in a fluid bed dryer wherein the inlet air has a water content in the range of 0.5 to 10 g per kg of air, or
wherein in step i.) tapentadol base is comprised in a reaction or diluted reaction mixture.

13. A process for the preparation of tapentadol maleate Form I or II or mixtures thereof by seeding the solution of tapentadol maleate with crystals of tapentadol maleate Form I or II or mixtures thereof,
tapentadol maleate Form I being **characterized by** the following 2-theta degrees: 11,5; 18,6; 22,1 ±0.2, and
tapentadol maleate Form II being **characterized by** the following 2-theta degrees: 17,1; 19,2; 19,9 ±0.2.

14. Use of crystalline tapentadol maleate, **characterized in** water content from 0 - 5 w/w % for the preparation of pharmaceutical dosage forms, said crystalline tapentadol maleate being selected from tapentadol maleate Form I and tapentadol maleate Form II,
tapentadol maleate Form I being **characterized by** the following 2-theta degrees: 11,5; 18,6; 22,1 ±0.2, and
tapentadol maleate Form II being **characterized by** the following 2-theta degrees: 17,1; 19,2; 19,9 ±0.2.

15. Use of crystalline tapentadol maleate according to claim 14, wherein the crystalline tapentadol maleate is tapentadol maleate Form II **characterized by** the following 2-theta degrees: 17,1; 19,2; 19,9 ±0.2.

## Patentansprüche

1. Kristallines Tapentadolmaleat, wobei das kristalline Tapentadolmaleat kristallines Tapentadolmaleat, **gekennzeichnet durch** die folgenden 2 Theta-Grade: 11,5; 18,6; 22,1 ±0,2, bevorzugt
ferner **gekennzeichnet durch** die folgenden 2 Theta-Grade: 11,5; 16,4; 18,1; 18,6; 19,8; 22,1 ±0,2, stärker bevorzugt
ferner **gekennzeichnet durch** die folgenden 2 Theta-Grade: 9,7; 11,5; 16,4; 18,1; 18,6; 19,8; 22,1; 23,3; 26,1 ±0,2, oder
kristallines Tapentadolmaleat, **gekennzeichnet durch** die folgenden 2 Theta-Grade: 17,1; 19,2; 19,9 ±0,2, bevorzugt
**gekennzeichnet durch** die folgenden 2 Theta-Grade: 16,9; 17,1; 19,2; 19,9; 22,8; 24,0 ±0,2, stärker bevorzugt
ferner **gekennzeichnet durch** die folgenden 2 Theta-Grade: 16,9; 17,1; 19,2; 19,9; 21,4; 22,8; 24,0; 27,3 ±0,2, insbesondere
ferner **gekennzeichnet durch** die folgenden 2 Theta-Grade: 11,1; 12,8; 16,9; 17,1; 19,2; 19,9; 21,4; 22,8; 24,0; 27,3 ±0,2, ist.

2. Kristallines Tapentadolmaleat gemäß Anspruch 1, **gekennzeichnet durch** einen Wassergehalt von 0 bis 5 % (w/w).

3. Kristallines Tapentadolmaleat gemäß Anspruch 1, **dadurch gekennzeichnet, dass** es eine hydratisierte oder wasserfreie kristalline Form von Tapentadolmaleat ist.

4. Kristallines Tapentadolmaleat gemäß Anspruch 1, **dadurch gekennzeichnet, dass** es kristallines Tapentadolmaleat, **gekennzeichnet durch** die folgenden 2 Theta-Grade: 17,1; 19,2; 19,9 ±0,2, bevorzugt
**gekennzeichnet durch** die folgenden 2 Theta-Grade: 16,9; 17,1; 19,2; 19,9; 22,8; 24,0 ±0,2, stärker bevorzugt
ferner **gekennzeichnet durch** die folgenden 2 Theta-Grade: 16,9; 17,1; 19,2; 19,9; 21,4; 22,8; 24,0; 27,3 ±0,2, insbesondere
ferner **gekennzeichnet durch** die folgenden 2 Theta-Grade: 11,1; 12,8; 16,9; 17,1; 19,2; 19,9; 21,4; 22,8; 24,0; 27,3 ±0,2, ist.

5. Kristallines Tapentadolmaleat gemäß Anspruch 4, welches kristallines Tapentadolmaleat-Hemihydrat ist.

6. Kristallines Tapentadolmaleat gemäß Anspruch 1, **dadurch gekennzeichnet, dass** es kristallines Tapentadolmaleat, **gekennzeichnet durch** die folgenden 2 Theta-Grade: 11,5; 18,6; 22,1 ±0,2, bevorzugt
ferner **gekennzeichnet durch** die folgenden 2 Theta-Grade: 11,5; 16,4; 18,1; 18,6; 19,8; 22,1 ±0,2, stärker bevorzugt
ferner **gekennzeichnet durch** die folgenden 2 Theta-Grade: 9,7; 11,5; 16,4; 18,1; 18,6; 19,8; 22,1; 23,3; 26,1 ±0,2, ist.

7. Gemisch von Tapentadolmaleat umfassend Tapentadolmaleat Form I und/oder Form II, wobei Tapentadolmaleat Form I durch die folgenden 2 Theta-Grade gekennzeichnet ist: 11,5; 18,6; 22,1 ±0,2, und Tapentadolmaleat Form II durch die folgenden 2 Theta-Grade gekennzeichnet ist: 17,1; 19,2; 19,9 ±0,2.

8. Gemisch von Tapentadolmaleat, wobei das Gemisch ein Gemisch von wasserfreien und hydratisierten kristallinen Formen von Tapentadolmaleat ist, wobei die wasserfreie Form Form I von Tapentadolmaleat ist, wobei Tapentadolmaleat Form I durch die folgenden 2 Theta-Grade gekennzeichnet ist: 11,5; 18,6; 22,1 ±0,2.

9. Verfahren zur Herstellung von kristallinem Tapentadolmaleat umfassend die folgenden Schritte:
i. Lösen von freier Base von Tapentadol in einem ersten Lösungsmittel, wobei das erste Lösungsmittel aus (C1-C6) Alkoholen, Nitrilen, halogenierten Kohlenwasserstoffen, zyklischen halogenierten oder nicht-halogenierten Kohlenwasserstoffen, Estern, Ketonen, Dimethylsulfoxid (DMSO), Dimethylformamid (DMF) und Gemischen davon ausgewählt ist,
ii. Zugeben von Maleinsäure zu der Bildung von Tapentadolmaleat-Lösung,
iii. Verdampfen des ersten Lösungsmittels, bis eine minimale Konzentration von 500 mg Tapentadolmaleat pro g Lösung erreicht ist,
iv. Zugeben von einem zweiten Lösungsmittel zu dem erhaltenen Rückstand nach dem Verdampfen,
v. Mischen des Kristallisationsgemisches bei Kristallisationstemperatur, bis eine Suspension gebildet wird,
vi. Isolieren und Trocknen des gebildeten Feststoffes.

10. Verfahren zur Herstellung von Tapentadolmaleat Form II aus Tapentadolmaleat Form I oder Anreichern von Gemischen von Tapentadolmaleat Form I und II durch Aufbringen von mechanischer Belastung auf Tapentadolmaleat, insbesondere durch Aufbringen von mechanischer Belastung auf Tapentadolmaleat Form I oder auf ein Gemisch umfassend oder bestehend aus Tapentadolmaleat Form I und II,
wobei Tapentadolmaleat Form I durch die folgenden 2 Theta-Grade gekennzeichnet ist: 11,5; 18,6; 22,1 ±0,2, und
Tapentadolmaleat Form II durch die folgenden 2 Theta-Grade gekennzeichnet ist: 17,1; 19,2; 19,9 ±0,2.

11. Verfahren gemäß Anspruch 10, wobei die mechanische Belastung durch Mahlen aufgebracht wird.

12. Verfahren gemäß Anspruch 9, wobei in Schritt vi.) das Trocknen von isoliertem Tapentadolmaleat in einem Fließbetttrockner durchgeführt wird,
wobei die Einlassluft einen Wassergehalt im Bereich von 0,5 bis 10 g pro kg Luft aufweist, oder
wobei in Schritt i.) Tapentadol-Base in einem Reaktions- oder verdünnten Reaktionsgemisch enthalten ist.

13. Verfahren zur Herstellung von Tapentadolmaleat Form I oder II oder Gemischen davon durch Animpfen der Lösung von Tapentadolmaleat mit Kristallen von Tapentadolmaleat Form I oder II oder Gemischen davon,
wobei Tapentadolmaleat Form I durch die folgenden 2 Theta-Grade gekennzeichnet ist: 11,5; 18,6; 22,1 ±0,2, und
Tapentadolmaleat Form II durch die folgenden 2 Theta-Grade gekennzeichnet ist: 17,1; 19,2; 19,9 ±0,2.

14. Verwendung von kristallinem Tapentadolmaleat, **gekennzeichnet durch** einen Wassergehalt von 0 - 5 % (w/w) zur Herstellung von pharmazeutischen Dosierungsformen, wobei das kristalline Tapentadolmaleat aus Tapentadolmaleat Form I und Tapentadolmaleat Form II ausgewählt ist,
wobei Tapentadolmaleat Form I durch die folgenden 2 Theta-Grade gekennzeichnet ist: 11,5; 18,6; 22,1 ±0,2, und
Tapentadolmaleat Form II durch die folgenden 2 Theta-Grade gekennzeichnet ist: 17,1; 19,2; 19,9 ±0,2.

15. Verwendung von kristallinem Tapentadolmaleat gemäß Anspruch 14, wobei das kristalline Tapentadolmaleat Tapentadolmaleat Form II, **gekennzeichnet durch** die folgenden 2 Theta-Grade: 17,1; 19,2; 19,9 ±0,2, ist.

## Revendications

1. Maléate de tapentadol cristallin, ledit maléate de tapentadol cristallin étant le maléate de tapentadol cristallin **caractérisé par** les degrés 2-thêta suivants : 11,5 ; 18,6 ; 22,1 ±0,2, de préférence
**caractérisé en outre par** les degrés 2-thêta suivants : 11,5 ; 16,4 ; 18,1 ; 18,6 ; 19,8 ; 22,1 ±0,2, plus préférablement
**caractérisé en outre par** les degrés 2-thêta suivants : 9,7 ; 11,5 ; 16,4 ; 18,1 ; 18,6 ; 19,8 ; 22,1 ; 23,3 ; 26,1 ±0,2, ou
le maléate de tapentadol cristallin **caractérisé par** les degrés 2-thêta suivants : 17,1 ; 19,2 ; 19,9 ±0,2, de préférence
**caractérisé par** les degrés 2-thêta suivants : 16,9 ; 17,1 ; 19,2 ; 19,9 ; 22,8 ; 24,0 ±0,2, plus préférablement
**caractérisé en outre par** les degrés 2-thêta suivants : 16,9 ; 17,1 ; 19,2 ; 19,9 ; 21,4 ; 22,8 ; 24,0 ; 27,3 ±0,2, en particulier
**caractérisé en outre par** les degrés 2-thêta suivants : 11,1 ; 12,8 ; 16,9 ; 17,1 ; 19,2 ; 19,9 ; 21,4 ; 22,8 ; 24,0 ; 27,3 ±0,2.

2. Maléate de tapentadol cristallin selon la revendication 1, **caractérisé par** une teneur en eau allant de 0 à 5 % poids.

3. Maléate de tapentadol cristallin selon la revendication 1, **caractérisé en ce qu'**il s'agit d'une forme cristalline hydratée ou anhydre de maléate de tapentadol.

4. Maléate de tapentadol cristallin selon la revendication 1, **caractérisé en ce qu'**il s'agit de maléate de tapentadol cristallin **caractérisé par** les degrés 2-thêta suivants : 17,1 ; 19,2 ; 19,9 ±0,2, de préférence
**caractérisé par** les degrés 2-thêta suivants : 16,9 ; 17,1 ; 19,2 ; 19,9 ; 22,8 ; 24,0 ±0,2, plus préférablement
**caractérisé en outre par** les degrés 2-thêta suivants : 16,9 ; 17,1 ; 19,2 ; 19,9 ; 21,4 ; 22,8 ; 24,0 ; 27,3 ±0,2, en particulier
**caractérisé en outre par** les degrés 2-thêta suivants : 11,1 ; 12,8 ; 16,9 ; 17,1 ; 19,2 ; 19,9 ; 21,4 ; 22,8 ; 24,0 ; 27,3 ±0,2.

5. Maléate de tapentadol cristallin selon la revendication 4, qui est le maléate de tapentadol cristallin semi-hydraté.

6. Maléate de tapentadol cristallin selon la revendication 1, **caractérisé en ce qu'**il s'agit de maléate de tapentadol cristallin **caractérisé par** les degrés 2-thêta suivants : 11,5 ; 18,6 ; 22,1 ±0,2, de préférence
**caractérisé en outre par** les degrés 2-thêta suivants : 11,5 ; 16,4 ; 18,1 ; 18,6 ; 19,8 ; 22,1 ±0,2, plus préférablement
**caractérisé en outre par** les degrés 2-thêta suivants : 9,7 ; 11,5 ; 16,4 ; 18,1 ; 18,6 ; 19,8 ; 22,1 ; 23,3 ; 26,1 ±0,2.

7. Mélange de maléate de tapentadol comprenant le maléate de tapentadol de Forme I et/ou de Forme II,
la Forme I de maléate de tapentadol étant **caractérisée par** les degrés 2-thêta suivants : 11,5 ; 18,6 ; 22,1 ±0,2, et
la Forme II de maléate de tapentadol étant **caractérisée par** les degrés 2-thêta suivants : 17,1 ; 19,2 ; 19,9 ±0,2.

8. Mélange de maléate de tapentadol, ledit mélange étant un mélange de formes cristallines anhydres et hydratées de maléate de tapentadol, la forme anhydre étant la Forme I de maléate de tapentadol, la Forme I de maléate de tapentadol étant **caractérisée par** les degrés 2-thêta suivants : 11,5 ; 18,6 ; 22,1 ±0,2.

9. Procédé de préparation de maléate de tapentadol cristallin comprenant les étapes suivantes consistant :
i. à dissoudre une base libre de tapentadol dans un premier solvant, le premier solvant étant choisi parmi les alcools en (C1 à C6), les nitriles, les hydrocarbures halogénés, les hydrocarbures cycliques halogénés ou non halogénés, les esters, les cétones, le diméthylsulfoxyde (DMSO), le diméthylformamide (DMF) et des mélanges de ceux-ci,
ii. à ajouter l'acide maléique pour la formation d'une solution de maléate de tapentadol,
iii. à évaporer le premier solvant jusqu'à l'obtention d'une concentration minimale de 500 mg de maléate de tapentadol par g de solution,
iv. à ajouter un deuxième solvant au résidu obtenu après évaporation,
v. à mélanger le mélange de cristallisation à la température de cristallisation jusqu'à la formation d'une suspension,
vi. à isoler et sécher le solide formé.

10. Procédé de préparation de la Forme II de maléate tapentadol à partir de la Forme I de maléate de tapentadol ou de mélanges enrichissants des Formes I et II de maléate de tapentadol en appliquant une contrainte mécanique au maléate de tapentadol, en particulier en appliquant une contrainte mécanique à la Forme I de maléate de tapentadol ou à un mélange comprenant ou consistant en Formes I et II de maléate de tapentadol,
la Forme I de maléate de tapentadol étant **caractérisée par** les degrés 2-thêta suivants : 11,5 ; 18,6 ; 22,1 ±0,2 et
la Forme II de maléate de tapentadol étant **caractérisée par** les degrés 2-thêta suivants : 17,1 ; 19,2 ; 19,9 ±0,2.

11. Procédé selon la revendication 10, dans lequel la contrainte mécanique est appliquée par meulage.

12. Procédé selon la revendication 9, dans lequel dans l'étape vi) le séchage du maléate de tapentadol isolé est effectué dans un séchoir à lit fluidisé dans lequel l'air d'entrée a une teneur en eau se trouvant dans la plage allant de 0,5 à 10 g par kg d'air, ou
dans lequel dans l'étape i) la base de tapentadol est comprise dans un mélange réactionnel ou un mélange réactionnel dilué.

13. Procédé de préparation de la Forme I ou II de maléate de tapentadol ou de mélanges de celles-ci par ensemencement de la solution de maléate de tapentadol avec des cristaux de la Forme I ou II de maléate de tapentadol ou des mélanges de celles-ci,
la Forme I de maléate de tapentadol étant **caractérisée par** les degrés 2-thêta suivants : 11,5 ; 18,6 ; 22,1 ±0,2 et
la Forme II de maléate de tapentadol étant **caractérisée par** les degrés 2-thêta suivants : 17,1 ; 19,2 ; 19,9 ±0,2,

14. Utilisation du maléate de tapentadol cristallin, **caractérisé par** une teneur en eau allant de 0 à 5 % poids pour la préparation de formes galéniques pharmaceutiques, ledit maléate de tapentadol cristallin étant choisi parmi la Forme I de maléate de tapentadol et la Forme II de maléate de tapentadol,
la Forme I de maléate de tapentadol étant **caractérisée par** les degrés 2-thêta suivants : 11,5 ; 18,6 ; 22,1 ±0,2 et
la Forme II de maléate de tapentadol étant **caractérisée par** les degrés 2-thêta suivants : 17,1 ; 19,2 ; 19,9 ±0,2.

15. Utilisation du maléate de tapentadol cristallin selon la revendication 14, dans laquelle le maléate de tapentadol cristallin est la Forme II de maléate de tapentadol **caractérisée par** les degrés 2-thêta suivants : 17,1 ; 19,2 ; 19,9 ±0,2.
